# EUROPEAN PATENT APPLICATION

(11) **EP 3 868 215 A1**
(43) Date of publication of application: **25.08.2021**
(21) Application number: 19872921.2
(22) Date of filing: 16.10.2019
(51) Int. Cl.: A23L 33/10, A61K 31/194, A61P 25/20

(54) **AGENT FOR IMPROVING QUALITY OF SLEEP**

(30) Priority: 16.10.2018 JP 2018195043; 17.10.2018 JP 2018195570; 19.02.2019 JP 2019027414
(71) Applicant: Nippon Chemiphar Co., Ltd., Tokyo 101-0032 (JP)
(72) Inventor: KAWAGUCHI Kazuhiko, Tokyo 101-0032 (JP); YAMASAKI Satomi, Tokyo 101-0032 (JP)
(74) Representative: Beckmann, Claus
(86) International application number: PCT/JP2019/040639
(87) International publication number: WO 2020/080394

(57) **Abstract**

The present invention provides a food composition or pharmaceutical composition which includes, as an active ingredient, at least one substance selected from the group consisting of citric acid and a salt thereof, or sodium bicarbonate. Ingestion or administration of the food composition or pharmaceutical composition leads to an improvement in quality of sleep.

## Description

### Technical Field

The present invention relates to an agent for improving quality of sleep (e.g., a food composition or pharmaceutical composition) that includes, as an active ingredient, citric acid or a salt thereof, or sodium bicarbonate (also referred to as "baking soda"). Further, the present invention relates to a composition for increasing a blood ornithine level, a blood serotonin level, a blood taurine level, a blood cystine level, or a blood nicotine amide level that includes, as an active ingredient, citric acid or a salt thereof, or sodium bicarbonate (e.g., a food composition or pharmaceutical composition).

This application claims priority based on Japanese Patent Application No. 2018-195043 filed in Japan on October 16, 2018, Japanese Patent Application No. 2018-195570 filed in Japan on October 17, 2018, and Japanese Patent Application No. 2019-27414 filed in Japan on February 19, 2019, and their contents are incorporated herein by reference.

### Background Art

Sleep is deeply involved in mental and physical rest, physical growth, repair or metabolism, or memory reconstruction, and it is necessary to obtain appropriate sleep in order to lead a healthy life. However, for example, according to the "2016 National Health and Nutrition Survey" of the Ministry of Health, Labor and Welfare of Japan, the percentage of those who do not get enough rest with sleep is 19.7%, and the percentage has been significantly increasing in recent years. Further, the percentage of people in their 20s to 50s exceeds 20%. It is not always easy to secure sufficient sleep duration due to various circumstances of the living environment, and it is important to improve the quality of sleep in order to obtain very satisfying sleep in a regular sleep period.

Ornithine is one of the amino acids and is known as a substance constituting the ornithine cycle that converts harmful ammonia into urea in the living body. Recently, it has been reported that orally administered ornithine decreases the blood cortisol level and improves the quality of sleep (Non Patent Literature 1). Further, it is expected that ornithine reduces the feeling of fatigue and improve the quality of sleep because of ammonia detoxification that converts ammonia into urea (hereinafter, sometimes simply referred to as "ammonia detoxification").

Serotonin is synthesized in vivo from tryptophan through 5-hydroxytryptophan. It is known that most of serotonin is distributed in the mucous membrane of the digestive tract and acts on the peristaltic movement of the intestine, but it also exists as a neurotransmitter in the brain, and is responsible for physiological functions such as biological rhythm, sleep or thermoregulation, and psychological functions such as mental stabilization. Further, it is known that serotonin secreted during the day is converted to melatonin from evening to midnight, and such melatonin promotes falling asleep. It has been reported that the deficiency of serotonin impairs a sleep-wake rhythm (Non Patent Literature 2), and the ingestion of tryptophan, i.e., a precursor of serotonin, is useful in improving the sleep-wake rhythm (Non Patent Literature 3).

Taurine (also referred to as "2-aminoethanesulfonic acid") is one of the sulfur-containing amino acid-like substances existing in the living body, and is known to be involved in the maintenance of homeostasis in vivo. Further, since taurine has the effect of recovering from brain fatigue, recovering from mental fatigue, or recovering from physical fatigue due to repair of liver function, it is expected to reduce the feeling of fatigue and improve the quality of sleep.

On the other hand, alcohol (particularly ethanol) is known to affect the physiological and psychological states, and the ingestion of relatively small amounts of alcoholic beverages may also contribute to the improvement of the quality of sleep, for example, it allows people to be in a cheerful mood or decreases the time to onset of sleep. However, the ingestion of excessive alcoholic beverages is known to cause so-called or alcohol sickness or hangover condition accompanied by nausea, vomiting, headache, or dizziness. Ingested alcohol is metabolized to acetic acid via acetaldehyde in vivo and further decomposed into carbon dioxide and water, but it is understood that acetaldehyde produced by the metabolism of alcohol is deeply involved in alcohol sickness or hangover. Aldehyde dehydrogenase 2 is an enzyme that metabolizes acetaldehyde to acetic acid in vivo. It is known that the percentage of Japanese who do not have normal aldehyde dehydrogenase 2 is higher than that of Westerners. Individuals who do not have aldehyde dehydrogenase 2 are classified as "flushers" and are prone to cause flushing reactions (such as flushing, palpitation, nausea, and hypotension) when they drink small amounts of alcohol.

Ornithine has an effect of improving liver function and an effect of reducing the feeling of fatigue due to the above-described ammonia detoxification, and is thus expected to be effective in suppressing alcohol sickness or hangover due to alcoholic beverage ingestion. Further, it has been reported that ornithine orally administered causes a decrease in salivary cortisol and an improvement in feeling of fatigue the next morning after flushers drank alcohol (Non Patent Literature 4).

It is known that cystine (also referred to as "3,3'-dithiobis(2-aminopropionic acid)") is one of the amino acids, and is contained in a large amount as a constituent of keratin that constitutes hair or nails. Further, cystine is known to have the effect of promoting the metabolism of acetaldehyde in vivo.

Nicotinamide (also referred to as "niacin or vitamin B₃") is known as a constituent of oxidoreductase coenzymes: nicotinamide adenine dinucleotide (abbreviated as: NAD) and nicotinamide adenine dinucleotide phosphate (abbreviated as: NADP) in vivo. These coenzymes function as electron acceptors or hydrogen donors for redox reactions in vivo. These coenzymes also function as coenzymes for alcohol dehydrogenase and acetaldehyde dehydrogenase, which are alcohol-metabolizing enzymes, and thus play an important role in alcohol metabolism. Nicotinamide is biosynthesized from tryptophan in vivo. Tryptophan is also a biosynthetic raw material for serotonin and melatonin. Accordingly, when nicotine amide is consumed by alcohol metabolism, tryptophan is consumed for nicotinamide biosynthesis to supplement it, and the proportion of tryptophan used for serotonin or melatonin biosynthesis is decreased. As a result, a shortage of serotonin or melatonin may adversely affect the quality of sleep. Therefore, the ingestion of nicotinamide is expected to contribute to the promotion of alcohol and acetaldehyde metabolism and the suppression of deterioration of quality of sleep during alcohol metabolism.

Nocturnal polyuria is observed in 30 to 50% of the elderly individuals and is a major cause of a symptom called "nocturia", in which the elderly individuals need to urinate during their night-time sleep and have to get up at least once to urinate. In fact, nocturnal polyuria is found in 80% of patients with nocturia (Non Patent Literature 5).

It is said that nocturia is a highly disturbing symptom in daily life, and the symptom causes nocturnal awakening due to urge to urinate, deteriorates the quality of sleep, causes daytime sleepiness, and affects work and housework. In particular, when the frequency of urination during sleep is 2 or more, the quality of life (QOL) deteriorates, which is often the target of treatment.

As described above, nocturnal polyuria is the main cause of nocturia, and decreased secretion of nocturnal antidiuretic hormone: arginine vasopressin (AVP) in the elderly individuals is considered to be one of the causes of an increase in nocturnal urine output (Non Patent Literature 6). Accordingly, stimulation of the V2 receptor, which is an AVP receptor and exerts an antidiuretic effect, is expected to lead to improvement in nocturnal polyuria and to improvement in nocturia. In fact, desmopressin (dDAVP), i.e., the V2 receptor-selective agonist, has been reported to reduce the nocturnal urine output and the frequency of nocturnal urination and increase the initial sleep time (Non Patent Literatures 7 and 8).

However, the V2 receptor agonist theoretically promotes fluid retention and there is concern about hyponatremia. Therefore, it has been reported that when the V2 receptor agonist is administered to the elderly individuals, who account for the majority of patients with nocturnal polyuria and patients with nocturia, caution should be exercised, such as measurement of serum sodium level (Patent Literature 1).

Incidentally, citric acid is known to be useful as an additive such as a stabilizer, a buffer, or a flavoring agent in the field of medical drugs or foods.

It is known that the administration of a salt of citric acid, particularly an alkali metal salt, or sodium bicarbonate (also referred to as "baking soda") (hereinafter, these may be collectively referred to as "alkalizing agent") to a patient with hyperuricemia or a patient with gout is useful in suppressing the formation of urinary stones in these patients. Further, it is known that since the alkalizing agent has an effect of making the body fluid of a patient alkaline, it is effective in improving the acidosis of the patient, particularly the metabolic acidosis such as renal tubular acidosis.

However, it is not known that citric acid or a salt thereof, or sodium bicarbonate has an effect of increasing a blood ornithine level, a blood serotonin level, a blood taurine level, a blood cystine level, or a blood nicotine amide level in mammals (particularly humans). Moreover, it is not known that citric acid or a salt thereof, or sodium bicarbonate is useful in improving the quality of sleep in mammals (particularly humans).

Sleep is classified into REM sleep and non-REM sleep (NREM sleep) according to the brain wave pattern, and NREM sleep is further divided into four stages; stage 1, stage 2, stage 3, and stage 4 in ascending order according to the level of sleep. Deep sleep in stages 3 and 4 is called "slow-wave sleep (or deep sleep)". In recent years, sleep debt, which is a state in which sleep deprivation has accumulated, has become a problem. In order to eliminate sleep debt, there is a need for procedure to increase the sleep duration and improve the quality of sleep.

### Citation List

### Patent Literature

Patent Literature 1: WO 2016/143200 A

### Non Patent Literature

Non Patent Literature 1: Miyake et al., Nutrition Journal 2014, 13:53
Non Patent Literature 2: Smaranda Leu-Semenescu et al., SLEEP, Vol. 33, No. 3, 2010, pp. 304-S1
Non Patent Literature 3: R. Bravo et al., AGE (2013) 35, pp. 1277-1285
Non Patent Literature 4: Kokubo et al., BioPsychoSocial Medicine 2013, 7: 6
Non Patent Literature 5: J. Urol., 2011; 186: 1358-1363
Non Patent Literature 6: Drugs Aging, 1999; 15: 429-437
Non Patent Literature 7: J. Urol., 2013; 190: 958-964
Non Patent Literature 8: J. Urol., 2013; 190: 965-972

### Summary of Invention

### Technical Problem

One of the objects of the present invention is to provide an agent for improving quality of sleep in mammals (particularly humans). Another object of the present invention is to provide a food composition or pharmaceutical composition for increasing a blood ornithine level, a blood serotonin level, a blood taurine level, a blood cystine level, or a blood nicotine amide level in mammals (particularly humans). Another object of the present invention is to provide a pharmaceutical composition useful in treating or preventing sleep disorders associated with frequent urination (e.g., nocturia, or nocturia due to nocturnal polyuria). Another object of the present invention is to provide a food composition or pharmaceutical composition useful in suppressing awakening after sleep onset.

### Solution to Problem

The present inventors have conducted diligent studies to achieve the above objects, and found that citric acid or a salt thereof, or sodium bicarbonate is useful in improving the quality of sleep in mammals (particularly humans), and completed the present invention.

The present invention has the following aspects:
(1) An agent for improving quality of sleep in mammals (particularly humans) including, as an active ingredient, citric acid or a salt thereof, or sodium bicarbonate;
(2) The agent for improving quality of sleep according to (1) including, as an active ingredient, at least one substance selected from the group consisting of citric acid and a salt thereof;
(3) The agent for improving quality of sleep according to (1) or (2), wherein the salt of citric acid is an alkali metal salt of citric acid;
(4) The agent for improving quality of sleep according to any one of (1) to (3), wherein the salt of citric acid is sodium citrate or a hydrate thereof, or potassium citrate or a hydrate thereof;
(5) The agent for improving quality of sleep according to any one of (1) to (4), wherein the improvement in quality of sleep is at least one selected from the group consisting of suppression of awakening after sleep onset (e.g., suppression of early-morning awakening (e.g., awakening after sleep onset 2 hours before waking up)) or suppression of awakening after sleep onset associated with frequent urination (e.g., nocturia, or nocturia due to nocturnal polyuria), improvement in sound sleep feeling, promotion of deep sleep, promotion of slow-wave sleep (e.g., increase in percentage of duration of slow-wave sleep (e.g., NREM sleep stage 3) to duration of total sleep, increase in duration of slow-wave sleep (e.g., NREM sleep stage 3), promotion of slow-wave sleep that decreases the percentage of duration of stage 1 to duration of total sleep, and increases the percentage of duration of stage 3 to duration of total sleep, or promotion of slow-wave sleep that reduces the duration of stage 1 and increases the duration of stage 3), and suppression of sleep disorders associated with frequent urination (e.g., nocturia, or nocturia due to nocturnal polyuria);
(6) The agent for improving quality of sleep according to any one of (1) to (5), wherein the improvement in quality of sleep is an improvement in quality of sleep in a first sleep cycle (e.g., an increase in delta power in the first sleep cycle or promotion of deep sleep in the first sleep cycle);
(7) The agent for improving quality of sleep according to any one of (1) to (5), wherein the improvement in quality of sleep is suppression in awakening after sleep onset associated with frequent urination (e.g., nocturia, or nocturia due to nocturnal polyuria) or suppression in sleep disorders associated with frequent urination (e.g., nocturia, or nocturia due to nocturnal polyuria);
(8) The agent for improving quality of sleep according to any one of (1) to (7), wherein the agent is a food composition whose package, container, or instruction indicates an improvement effect in quality of sleep (e.g., increase in percentage of duration of slow-wave sleep (e.g., NREM sleep stage 3) to duration of total sleep, increase in duration of slow-wave sleep (e.g., NREM sleep stage 3), promotion of slow-wave sleep that decreases the percentage of duration of stage 1 to duration of total sleep, and increases the percentage of duration of stage 3 to duration of total sleep, or promotion of slow-wave sleep that reduces the duration of stage 1 and increases the duration of stage 3)), a decrease in frequency of awakening after sleep onset (e.g., early-morning awakening (awakening after sleep onset 2 hours before waking up) or awakening after sleep onset associated with frequent urination (e.g., nocturia, or nocturia due to nocturnal polyuria), an improvement in sound sleep feeling, promotion of deep sleep, an improvement in quality of sleep in the first sleep cycle (e.g., increase in delta power in first sleep cycle, or promotion of deep sleep in the first sleep cycle) or an effect of suppressing sleep disorders associated with frequent urination (e.g., nocturia, or nocturia due to nocturnal polyuria));
(9) The agent for improving quality of sleep according to any one of (1) to (8), wherein the agent is a food composition or pharmaceutical composition;
(10) A food composition which is in a unit package form per ingestion (e.g., a unit package form per serving) and contains 500 mg or more of at least one substance selected from the group consisting of citric acid and a salt thereof;
(11) The agent for improving quality of sleep according to any one of (1) to (9), wherein the agent is in a unit package form per ingestion (e.g., a unit package form per serving), and is a food composition containing 500 mg or more of at least one substance selected from the group consisting of citric acid and a salt thereof;
(12) A composition for increasing a blood ornithine level, a blood serotonin level, a blood taurine level, a blood cystine level, or a blood nicotine amide level in mammals (particularly humans) that includes, as an active ingredient, citric acid or a salt thereof, or sodium bicarbonate (e.g., a food composition or pharmaceutical composition);
(13) The composition according to (12) including, as an active ingredient, at least one substance selected from the group consisting of citric acid and a salt thereof (e.g., a food composition or pharmaceutical composition);
(14) The agent for improving quality of sleep or composition according to any one of (1) to (13), including, as an active ingredient, citric acid, sodium citrate, and potassium citrate (e.g., a food composition or pharmaceutical composition); and
(15) The agent for improving quality of sleep or composition according to any one of (1) to (14), including, as an active ingredient, citric acid, sodium citrate, and potassium citrate at a molar ratio of 1 : 2 : 2 (e.g., a food composition or pharmaceutical composition).

The present invention has the following other aspects:
(1') An agent for improving quality of sleep in mammals including, as an active ingredient, at least one substance selected from the group consisting of citric acid and a salt thereof;
(2') The agent for improving quality of sleep according to (1') including citric acid as an active ingredient;
(3') The agent for improving quality of sleep according to (1') or (2') including, as an active ingredient, an alkali metal salt of citric acid;
(4') The agent for improving quality of sleep according to any one of (1') to (3') including, as an active ingredient, citric acid, sodium citrate or a hydrate thereof, and potassium citrate or a hydrate thereof;
(5') The agent for improving quality of sleep according to any one of (1') to (4'), wherein an improvement in quality of sleep is suppression in awakening after sleep onset;
(6') The agent for improving quality of sleep according to any one of (1') to (5'), wherein the improvement in quality of sleep is suppression in early-morning awakening;
(7') The agent for improving quality of sleep according to any one of (1') to (6'), wherein the improvement in quality of sleep is an improvement in sound sleep feeling;
(8') The agent for improving quality of sleep according to any one of (1') to (7'), wherein the improvement in quality of sleep is a promotion of slow-wave sleep;
(9') The agent for improving quality of sleep according to any one of (1') to (8'), wherein the improvement in quality of sleep is an increase in percentage of duration of NREM sleep stage 3 to duration of total sleep;
(10') The agent for improving quality of sleep according to any one of (1') to (9'), wherein the improvement in quality of sleep is a promotion of slow-wave sleep, which reduces a percentage of duration of NREM sleep stage 1 and increases a percentage of duration of NREM sleep stage 3;
(11') The agent for improving quality of sleep according to any one of (1') to (10'), wherein the improvement in quality of sleep is an improvement in quality of sleep in a first sleep cycle;
(12') The agent for improving quality of sleep according to any one of (1') to (11'), wherein the improvement in quality of sleep is an increase in delta power in the first sleep cycle;
(13') The agent for improving quality of sleep according to any one of (1') to (12'), wherein the improvement in quality of sleep is a promotion of deep sleep in the first sleep cycle;
(14') The agent for improving quality of sleep according to any one of (1') to (13'), wherein the improvement in quality of sleep is suppression of sleep disorders associated with frequent urination (e.g., nocturia, or nocturia due to nocturnal polyuria);
(15') The agent for improving quality of sleep according to any one of (1') to (14'), wherein the improvement in quality of sleep is suppression in awakening after sleep onset associated with frequent urination (e.g., nocturia, or nocturia due to nocturnal polyuria);
(16') The agent for improving quality of sleep according to any one of (1') to (15'), wherein the agent is a food product;
(17') The agent for improving quality of sleep according to any one of (1') to (16'), wherein the agent is in a unit package form per serving, and is a food product containing 500 mg or more of at least one substance selected from the group consisting of citric acid and a salt thereof;
(18') The agent for improving quality of sleep according to any one of (1') to (16'), wherein the agent is in a unit package form per serving, and is a food product containing 500 mg or more of at least one substance selected from the group consisting of citric acid and a salt thereof;
(19') The agent for improving quality of sleep according to any one of (1') to (18'), wherein the agent is a food product whose package, container, or instruction indicates an effect of improving the quality of sleep; and
(20') The agent for improving quality of sleep according to any one of (1') to (19'), wherein the agent is a food product whose package, container, or instruction indicates an effect of increasing a percentage of duration of slow-wave sleep to duration of total sleep, reducing a frequency of awakening after sleep onset, or improving sound sleep feeling.

### Advantageous Effects of Invention

The ingestion or administration of the composition provided by the present invention leads to an increase in blood ornithine level, an increase in blood serotonin level, an increase in blood taurine level, an increase in blood cystine level, or an increase in blood nicotine amide level in mammals (particularly humans). The ingestion or administration of the composition provided by the present invention leads to suppression of frequent urination (e.g., nocturia or nocturia due to nocturnal polyuria). The composition provided by the present invention is useful in improving the quality of sleep in mammals (particularly humans).

### Brief Description of Drawings

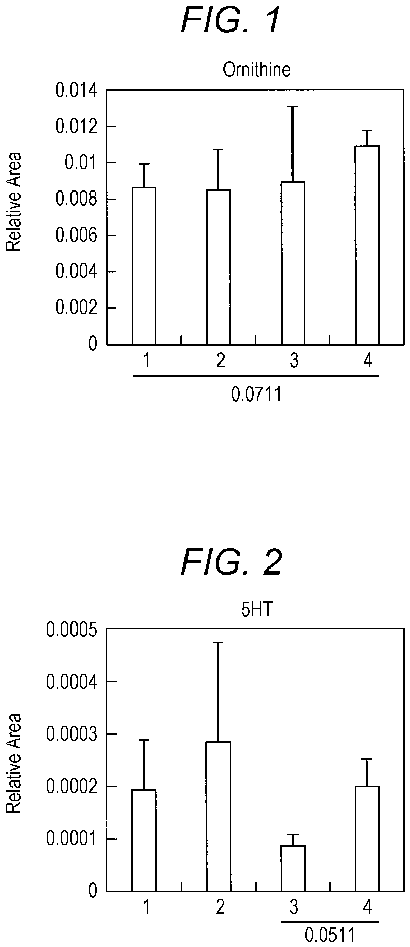

Fig. 1 is a graph illustrating changes in plasma ornithine level when a combination drug containing citric acid, potassium citrate monohydrate, and sodium citrate dihydrate has been administered to miniature pigs with renal failure. The graphs 1, 2, 3, and 4 on the horizontal axis show the results before administration, after the first week of administration, after the second week of discontinuation of administration, and after the third week of administration, respectively, and the vertical axis shows the relative area value of each test substance amount (the same applies to Figs. 2 to 5).
Fig. 2 is a graph illustrating changes in plasma serotonin level when a combination drug containing citric acid, potassium citrate monohydrate, and sodium citrate dihydrate has been administered to miniature pigs with renal failure.

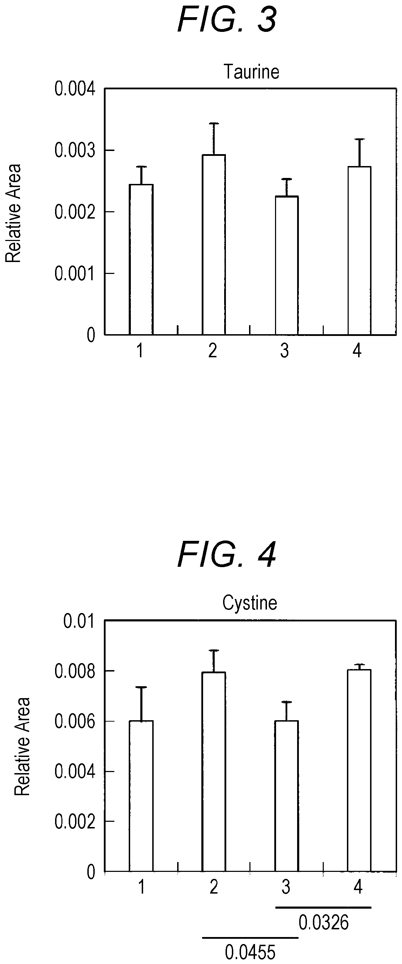

Fig. 3 is a graph illustrating changes in plasma taurine level when a combination drug containing citric acid, potassium citrate monohydrate, and sodium citrate dihydrate has been administered to miniature pigs with renal failure.
Fig. 4 is a graph illustrating changes in plasma cystine level when a combination drug containing citric acid, potassium citrate monohydrate, and sodium citrate dihydrate has been administered to miniature pigs with renal failure.

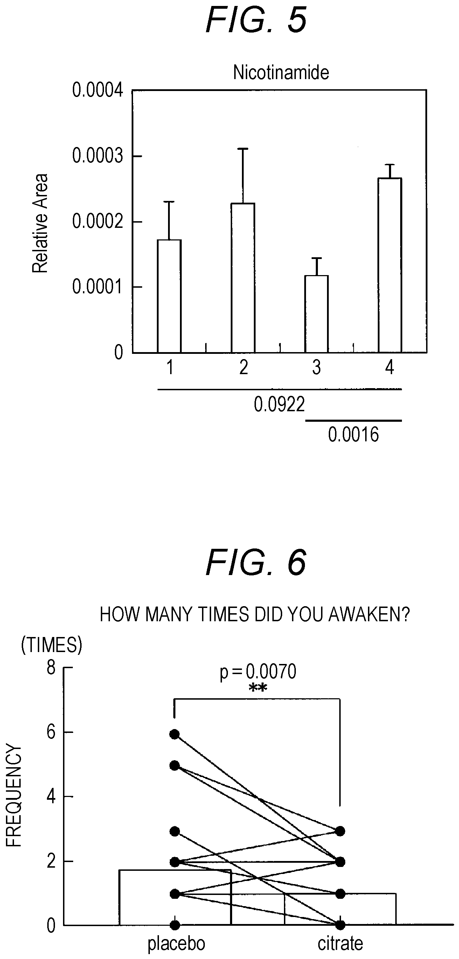

Fig. 5 is a graph illustrating changes in plasma nicotinamide level when a combination drug containing citric acid, potassium citrate monohydrate, and sodium citrate dihydrate has been administered to miniature pigs with renal failure.
Fig. 6 is a graph illustrating the results of evaluation of quality of sleep when a combination drug containing citric acid, potassium citrate monohydrate and sodium citrate dihydrate, or a placebo has been administered to subjects based on the St. Mary's Hospital Sleep Questionnaire (the results of evaluating how many times they have awakened).

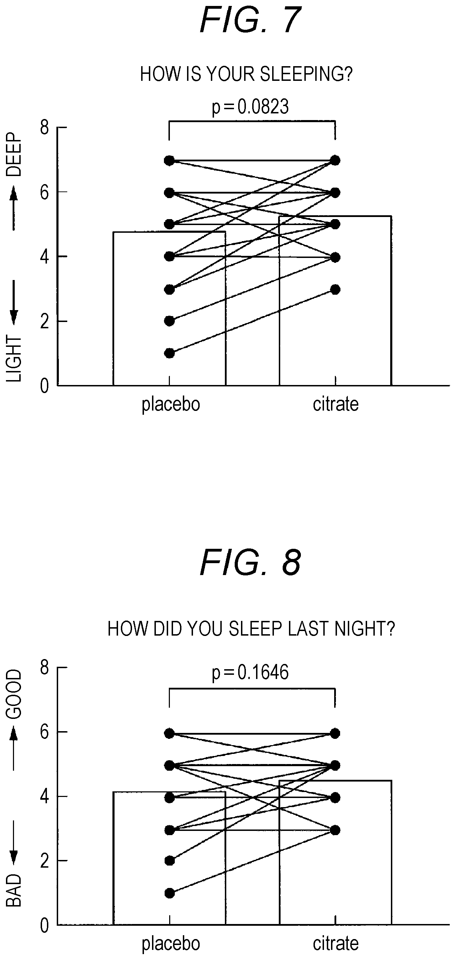

Fig. 7 is a graph illustrating the results of evaluation of quality of sleep when a combination drug containing citric acid, potassium citrate monohydrate and sodium citrate dihydrate, or a placebo has been administered to subjects based on the St. Mary's Hospital Sleep Questionnaire (the results of evaluating sleep).
Fig. 8 is a graph illustrating the results of evaluation of quality of sleep when a combination drug containing citric acid, potassium citrate monohydrate and sodium citrate dihydrate, or a placebo has been administered to subjects based on the St. Mary's Hospital Sleep Questionnaire (the results of evaluating how well they have slept).

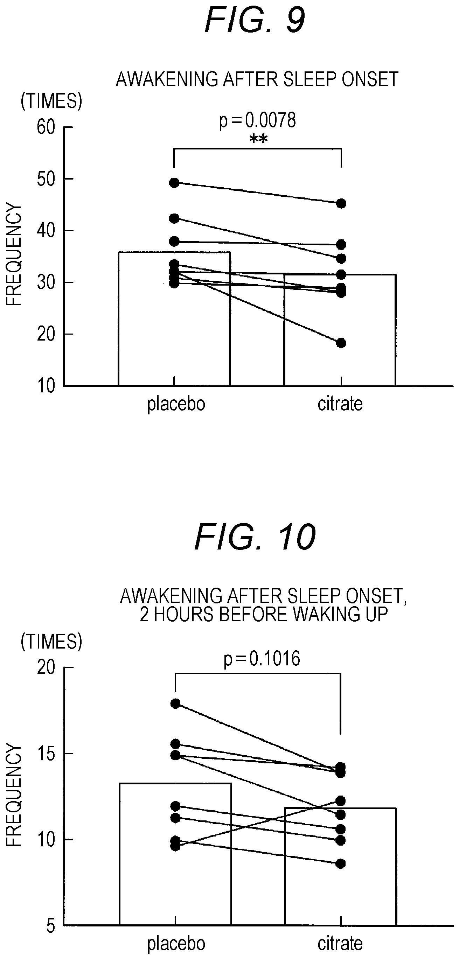

Fig. 9 is a graph illustrating the results of evaluation of quality of sleep when a combination drug containing citric acid, potassium citrate monohydrate and sodium citrate dihydrate, or a placebo has been administered to subjects based on brain wave measurement (the frequency of awakening after sleep onset).
Fig. 10 is a graph illustrating the results of evaluation of quality of sleep when a combination drug containing citric acid, potassium citrate monohydrate and sodium citrate dihydrate, or a placebo has been administered to subjects based on brain wave measurement (the frequency of awakenings after sleep onset, 2 hours before waking up).

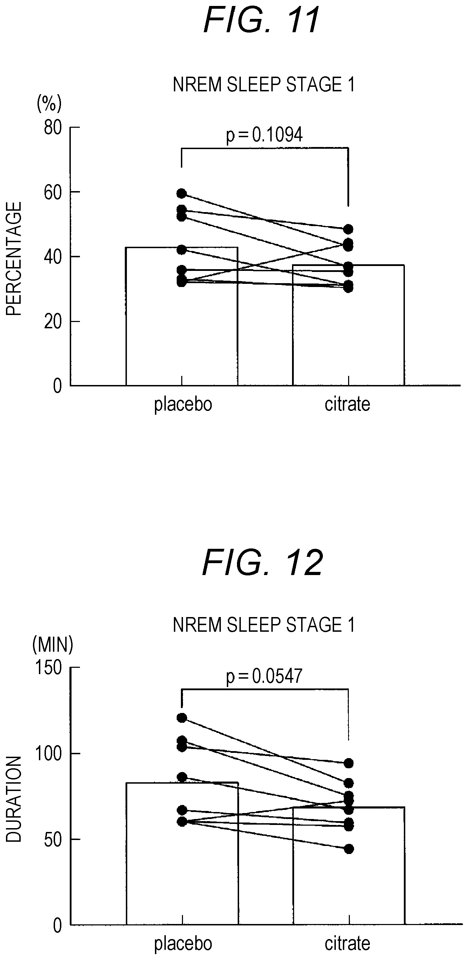

Fig. 11 is a graph illustrating the results of evaluation of quality of sleep when a combination drug containing citric acid, potassium citrate monohydrate and sodium citrate dihydrate, or a placebo has been administered to subjects based on brain wave measurement (percentage of duration of NREM sleep stage 1 to duration of total sleep).
Fig. 12 is a graph illustrating the results of evaluation of quality of sleep when a combination drug containing citric acid, potassium citrate monohydrate and sodium citrate dihydrate, or a placebo has been administered to subjects based on brain wave measurement (duration of NREM sleep stage 1).

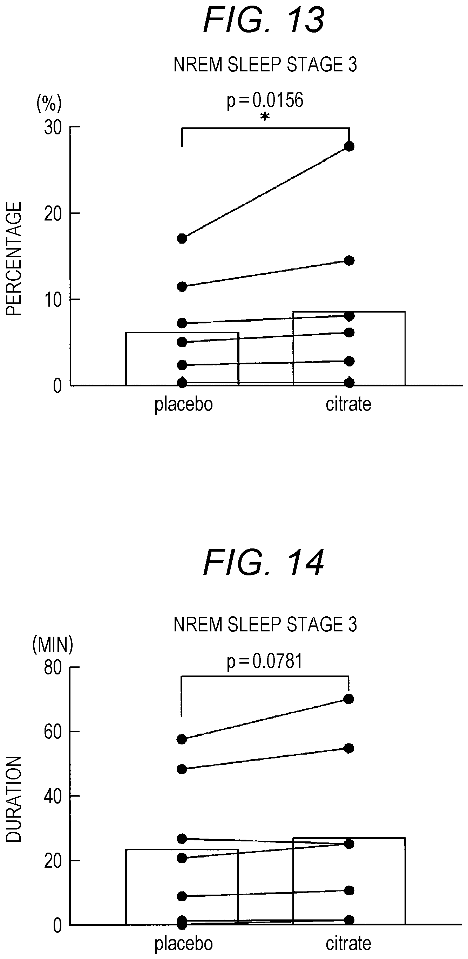

Fig. 13 is a graph illustrating the results of evaluation of quality of sleep when a combination drug containing citric acid, potassium citrate monohydrate and sodium citrate dihydrate, or a placebo has been administered to subjects based on brain wave measurement (percentage of duration of NREM sleep stage 3 to duration of total sleep).
Fig. 14 is a graph illustrating the results of evaluation of quality of sleep when a combination drug containing citric acid, potassium citrate monohydrate and sodium citrate dihydrate, or a placebo has been administered to subjects based on brain wave measurement (duration of NREM sleep stage 3).

### Description of Embodiments

The present invention provides an agent for improving quality of sleep which includes, as an active ingredient, citric acid or a salt thereof, or sodium bicarbonate (also referred to as "baking soda").

The agent for improving quality of sleep as provided by the present invention can be a food composition or pharmaceutical composition.

Further, the present invention provides a composition for increasing a blood ornithine level, a blood serotonin level, a blood taurine level, a blood cystine level, or a blood nicotine amide level which includes, as an active ingredient, citric acid or a salt thereof, or sodium bicarbonate. As provided by the present invention, the composition for increasing a blood ornithine level, a blood serotonin level, a blood taurine level, a blood cystine level, or a blood nicotine amide level can be a food composition or a pharmaceutical composition.

### 1. Food composition

The food composition provided by the present invention may include, as an active ingredient, citric acid or a salt thereof, or sodium bicarbonate.

In one embodiment, the food composition provided by the present invention may include, as an active ingredient, at least one substance selected from the group consisting of citric acid and a salt of citric acid.

Examples of citric acid include citric acid hydrate (e.g., citric acid monohydrate (C₆H₈O₇·H₂O) ) and anhydrous citric acid.

As the salt of citric acid, an edible or pharmaceutically-acceptable salt for citric acid is preferable, and examples thereof include salts of citric acid with alkali metal, alkaline earth metal, iron, or ammonium ion. More specific examples thereof include monopotassium citrate, dipotassium citrate, tripotassium citrate (in the present specification, tripotassium citrate may simply be referred to as "potassium citrate"), monosodium citrate, disodium citrate, trisodium citrate (in the present specification, trisodium citrate may be simply referred to as "sodium citrate"), calcium citrate, magnesium citrate, ferrous citrate, ferric citrate, sodium ferrous citrate, and ferric ammonium citrate.

As the salt of citric acid, alkali metal salts of citric acid such as monopotassium citrate, dipotassium citrate, potassium citrate, monosodium citrate, disodium citrate, and sodium citrate are preferable. Among them, potassium citrate, sodium citrate, and the like are preferable.

In one embodiment, the salt of citric acid is an edible or pharmaceutically-acceptable salt other than ferrous citrate and ferric citrate.

The salt of citric acid may be a hydrate thereof, or a different salt or a mixture of the hydrate. More specifically, hydrates such as potassium citrate monohydrate (C₆H₅K₃O₇·H₂O) and sodium citrate dihydrate (C₆H₅Na₃O₇-2H₂O) and mixtures thereof can be exemplified. For example, when the alkali metal salt of citric acid as an active ingredient is a mixture of potassium citrate monohydrate (C₆H₅K₃O₇·H₂O) and sodium citrate dihydrate (C₆H₅Na₃O₇·2H₂O), those skilled in the art can appropriately set the mixing ratio of potassium citrate monohydrate (C₆H₅K₃O₇·H₂O) and sodium citrate dihydrate (C₆H₅Na₃O₇·2H₂O)-For example, the molar ratio of potassium citrate monohydrate to sodium citrate dihydrate may be in a range of 1 : 0.01 to 1 : 100. The mixing ratio may be about 1 : 1 in molar ratio.

In one embodiment, the mixing ratio of the number of moles of sodium salt of citric acid and the number of moles of potassium salt of citric acid in the mixture can be appropriately set by those skilled in the art, and is preferably in a range of 0.85 : 1.15 to 1.15 : 0.85, more preferably in a range of 0.90 : 1.10 to 1.10 : 0.90, more preferably in a range of 0.95 : 1.05 to 1.05 : 0.95, still more preferably in a range of 0.99 : 1.01 to 1.01 : 0.99, and particularly preferably 1 : 1.

In one embodiment, the mixing ratio of the number of moles of sodium citrate dihydrate (C₆H₅Na₃O₇·2H₂O) and the number of moles of potassium citrate monohydrate (C₆H₅K₃O₇·H₂O) in the mixture can be appropriately set by those skilled in the art, and is preferably in a range of 0.85 : 1.15 to 1.15 : 0.85, more preferably in a range of 0.90 : 1.10 to 1.10 : 0.90, more preferably in a range of 0.95 : 1.05 to 1.05 : 0.95, still more preferably in a range of 0.99 : 1.01 to 1.01 : 0.99, and particularly preferably 1 : 1.

When the active ingredient is a mixture of potassium citrate monohydrate (C₆H₅K₃O₇·H₂O), sodium citrate dihydrate (C₆H₅Na₃O₇·2H₂O), and anhydrous citric acid, the mixing ratio of potassium citrate monohydrate (C₆H₅K₃O₇·H₂O), sodium citrate dihydrate (C₆H₅Na₃O₇·2H₂O, and anhydrous citric acid in the mixture of potassium citrate monohydrate (C₆H₅K₃O₇·H₂O), sodium citrate dihydrate (C₆H₅Na₃O₇·2H₂O), and anhydrous citric acid can be appropriately set by those skilled in the art. For example, as for the molar ratio of potassium citrate monohydrate to sodium citrate dihydrate and anhydrous citric acid, the molar ratio of potassium citrate monohydrate to sodium citrate dihydrate may be in a range of 1 : 0.01 to 1 : 100, and the molar ratio of potassium citrate monohydrate to anhydrous citric acid may be in a range of 1 : 0.01 to 1 : 100. The mixing ratio may be about 2 : 2 : 1 in molar ratio.

In one embodiment, the mixing ratio of the number of moles of anhydrous citric acid, the number of moles of sodium salt of citric acid, and the number of moles of potassium salt of citric acid in the mixture can be appropriately set by those skilled in the art, and is preferably in a range of 1 : 1.7-2.3 : 1.7-2.3, more preferably in a range of 1 : 1.9-2.1 : 1.9-2.1, still more preferably in a range of 1 : 1.95-2.05 : 1.95-2.05, and particularly preferably 1 : 2 : 2.

In one embodiment, the mixing ratio of the number of moles of anhydrous citric acid, the number of moles of sodium citrate dihydrate (C₆H₅Na₃O₇·2H₂O, and the number of moles of potassium citrate monohydrate (C₆H₅K₃O₇·H₂O) in the mixture can be appropriately set by those skilled in the art, and is preferably in a range of 1 : 1.7-2.3 : 1.7-2.3, more preferably in a range of 1 : 1.9-2.1 : 1.9-2.1, still more preferably in a range of 1 : 1.95-2.05: 1.95-2.05, and particularly preferably 1 : 2 : 2.

Further, other examples of preferred alkali metal salts of citric acid include sodium citrate or hydrates thereof. For example, sodium citrate dihydrate (C₆H₅Na₃O₇·2H₂O) may be used.

Furthermore, other examples of preferred alkali metal salts of citric acid include potassium citrate or hydrates thereof. For example, potassium citrate monohydrate (C₆H₅K₃O₇·H₂O) may be used.

In one embodiment, the active ingredient contained in the food composition of the present invention may include a mixture of sodium citrate or a hydrate thereof and potassium citrate or a hydrate thereof.

In another embodiment, the active ingredient contained in the food composition of the present invention may be comprised of only a mixture of sodium citrate or a hydrate thereof and potassium citrate or a hydrate thereof.

In the present specification, when referring to the weight of citric acid and a salt thereof (e.g., potassium citrate monohydrate (C₆H₅K₃O₇·H₂O) and sodium citrate dihydrate (C₆H₅Na₃O₇·2H₂O, and sodium bicarbonate, the weight may be dry weight.

An alkali metal salt of citric acid or sodium bicarbonate, also referred to as "alkalizing agent", is also known as an agent capable of increasing the HCO₃⁻concentration and pH of mammalian (particularly human) body fluids, such as blood or urine.

In the present specification, the term "mammals" include animals classified as Mammalia, and humans are particularly preferable.

For example, it is known that human "sleep" usually transitions to each state: light sleep (stages 1 and 2 of NREM sleep); slow-wave sleep (also referred to as "deep sleep", stages 3 and 4 of NREM sleep) (light sleep and slow-wave sleep may be referred to as "NREM sleep"); and REM sleep. Further, it is also known that awakening may occur during sleep and then transition to the sleep state again (which may be referred to as "awakening after sleep onset").

The "quality of sleep" can be generally evaluated by, for example, an objective index such as frequency and duration of awakening after sleep onset, sleep efficiency, presence/absence of early-morning awakening, extended NREM sleep duration, time to enter NREM sleep and REM sleep modes, time to enter slow-wave sleep mode, frequency and duration of slow-wave sleep, percentage of duration of slow-wave sleep to duration of total sleep, sleep duration, latency to sleep onset (time to fall asleep) or delta power during NREM sleep (index of depth of sleep), or an index based on relatively subjective view of subjects such as good sleep, refreshing sleep, sound sleep feeling, dreams (e.g., whether or not you have a nightmare, the frequency of dreams, etc.), satisfaction of sleep or degree of daytime sleepiness. Further, in the present specification, the term "quality of sleep" may also include the concept of length of sleep duration. The reason is as described below. In the lifestyle of subjects to which the food composition of the present invention is applied, the time zone devoted to sleep (hereinafter, may be referred to as "sleep time zone") may be limited. However, it is not always possible to maintain the sleep state during the sleep time zone, and it can be considered that the length of the sleep state in the sleep time zone affects the "quality of sleep". Further, in the lifestyle of subjects to which the food composition of the present invention is applied, in an environment where the sleep time zone can be extended, the longer the duration of the sleep state, the higher the "quality of sleep" may be.

In the present specification, the term "sleep duration" may be a duration from sleep-onset time (i.e., the time of transition from awakening to any of light sleep, slow-wave sleep, or REM sleep) to the time of transition to awakening immediately before waking up or at the time of waking up (excluding awakening after sleep onset) (also referred to as "time of waking up"). Light sleep, slow-wave sleep, REM sleep, or awakening after sleep onset may occur singularly or multiple times during sleep. In the present specification, light sleep duration, slow wave sleep duration, and REM sleep duration may refer to the time of each time of each state occurring multiple times, or may refer to the total time of each time of each state.

The improvement in quality of sleep according to the present invention may be an improvement in quality of sleep during the entire sleep period or an improvement in quality of sleep during a part of the sleep period, for example, an improvement in quality of sleep in the first sleep cycle. During sleep, NREM sleep and REM sleep alternate. In the present specification, the term "sleep cycle" refers to the time duration from the end of one REM sleep period to the end of the next. The term "first sleep cycle" refers to the first sleep cycle after falling asleep.

For example, comparison among the pre-ingestion of the food composition of the present invention, the control, and the placebo is performed, and the "improvement in quality of sleep" provided by the present invention can be evaluated as suppression of awakening after sleep onset (e.g., decrease in frequency of awakening after sleep onset), promotion of slow-wave sleep (e.g., increase in duration of slow-wave sleep (e.g., NREM sleep stage 3), increase in percentage of duration of slow-wave sleep (e.g., NREM sleep stage 3) to duration of total sleep, increase in delta power in the first sleep cycle), suppression of early-morning awakening (e.g., decrease in frequency of awakening after sleep onset 2 hours before waking up), increase in duration of slow-wave sleep, increase in percentage of duration of slow-wave sleep to duration of total sleep, or promotion of deep sleep (e.g., increase in delta power (e.g., the delta power of the first sleep cycle)) or an improvement in sound sleep feeling.

The frequency of awakening after sleep onset, REM sleep, NREM sleep, slow-wave sleep, delta power, and the like can be evaluated by a known method, and can be evaluated, for example, by measuring brain waves using an electroencephalograph.

In one embodiment, the "improvement in quality of sleep" is an increase in percentage of duration of slow-wave sleep (e.g., NREM sleep stage 3) to duration of total sleep. When a state of slow-wave sleep (e.g., NREM sleep stage 3) appears multiple times during sleep, the duration of the slow-wave sleep (e.g., NREM sleep stage 3) can be the total duration of each state of the slow-wave sleep (e.g., NREM sleep stage 3) that appears multiple times.

In one embodiment, the percentage of duration of slow-wave sleep to duration of total sleep is preferably increased by a range of 10% to 50%, and is more preferably by a range of 15% to 35%, as compared to the pre-ingestion of the food composition of the present invention or the control or the placebo.

In one embodiment, the "improvement in quality of sleep" is a decrease in the frequency of awakening after sleep onset.

In one embodiment, as for the frequency of awakening after sleep onset; the frequency of awakening determined from the measured brain waves is preferably decreased by a range of 5 to 30%, and is more preferably decreased by a range of 10 to 20%, as compared to the pre-ingestion of the food composition of the present invention or the control or the placebo. Further, in one embodiment, the frequency of awakening after sleep onset may be evaluated by the frequency of awakening which has been actually recognized during the sleep period. It is preferable that the frequency of awakening after sleep onset is decreased by 1, as compared to the pre-ingestion of the food composition of the present invention or the control or the placebo, and it is particularly preferable that the frequency becomes 0.

In one embodiment, the "improvement in quality of sleep" is an improvement in sound sleep feeling, and the improvement in sound sleep feeling is compared to the pre-ingestion of the food composition of the present invention or the control or the placebo, and may be evaluated using subjective feeling of having slept well as an index.

In one embodiment, the "improvement in quality of sleep" is a promotion of deep sleep. The promotion of deep sleep is compared to the pre-ingestion of the food composition of the present invention or the control or the placebo, and may be evaluated using subjective feeling of having slept well as an index, or may be evaluated using an increase in delta power as an index.

In the present specification, the term "nocturnal polyuria" refers to a state in which the nocturnal urine output is increased; for example, in elderly individuals, the urine output during night-time sleep exceeds 33% of the total daily urine output, and in young individuals, the urine output during night-time sleep exceeds 20% of the total daily urine output. Nocturia is a symptom that the individuals have to wake up at least once to urinate during sleep. Nocturia may be due to nocturnal polyuria.

If the frequency of nocturnal urination is reduced, awakening after sleep onset may be suppressed. Awakening after sleep onset can cause sleep disorders and worsen the quality of sleep. Further, even if urination does not occur, when nocturnal urine production increases, individuals may have urge to urinate and awaken. This may cause sleep disorders, and worsen the quality of sleep.

Therefore, the present invention provides, in one embodiment, a food composition for suppressing sleep disorders associated with frequent urination (e.g., nocturia, or nocturia due to nocturnal polyuria). In one embodiment, the present invention provides a food composition for suppressing awakening after sleep onset. In one embodiment, the present invention provides a food composition for improving quality of sleep (e.g., a food composition for improving quality of sleep due to a reduced urge to urinate at night or a decrease in the frequency of nocturnal urination).

In one embodiment, the "decrease in frequency of urination" may be evaluated by comparing to the pre-ingestion of the food composition of the present invention or the control or the placebo.

In one embodiment, the ingestion of the food composition provided by the present invention exerts an effect, such as increased blood ornithine level, increased blood serotonin level, increased blood taurine level, increased blood cystine level, or increased blood nicotine amide level, as compared to the pre-ingestion or the control or the placebo. Accordingly, the food composition provided by the present invention is useful in improving the quality of sleep.

The content of citric acid or a salt thereof, or sodium bicarbonate in the food composition provided by the present invention can be appropriately determined depending on the type of food. Examples of food compositions include foods for specified health use, foods with functional claims, foods for hospital patients, and supplements. The form of these food compositions is not particularly limited as long as it contains citric acid or a salt thereof or sodium bicarbonate in an effective amount that exerts the above effect, and is orally ingestible. The food compositions may be in the form of a normal food or drink, or may be formulated and provided as a formulation suitable for oral administration, such as a tablet, a capsule, a suspension, a granule, a jelly, or a health drink. In the present specification, with respect to the constitution and production method of these formulations, the formulation technology known per se in the field of pharmaceutical formulation technology (hereinafter, may be referred to as "pharmaceutical field") can be applied to the formulation of the food composition provided by the present invention.

The formulation of the food composition provided by the present invention includes, as an active ingredient, citric acid or a salt thereof, or sodium bicarbonate (e.g., includes, as an active ingredient, at least one substance selected from the group consisting of citric acid and a salt thereof), and may be prepared by using the active ingredient as it is, or may be prepared by mixing the active ingredient with an edible carrier, such as an excipient (e.g., lactose, D-mannitol, crystalline cellulose, or glucose), a binder (e.g., hydroxypropylcellulose (HPC), gelatin or polyvinylpyrrolidone (PVP)), a lubricant (e.g., magnesium stearate or talc), a disintegrant (e.g., starch or carboxymethylcellulose calcium (CMC-Ca)), a diluent (e.g., water or physiological saline), and, if necessary, other additives (e.g., a pH adjuster, a surfactant, a solubilizing agent, a preservative, an emulsifier, an isotonic agent, or a stabilizer). The dosage form of the formulation of the food composition provided by the present invention may be a formulation that can be orally ingested, such as tablets, capsules, suspensions, granules, jellies, and health drinks. For example, in order to make tablets, citric acid or a salt thereof, or sodium bicarbonate may be mixed with an excipient (e.g., lactose, D-mannitol, crystalline cellulose or glucose), a disintegrant (e.g., starch or carboxymethylcellulose calcium (CMC)-Ca)), a binder (e.g., hydroxypropylcellulose (HPC), gelatin, or polyvinylpyrrolidone (PVP)), a lubricant (e.g., magnesium stearate or talc), and the like for formulation. In the present specification, when the active ingredient of the food composition is citric acid or a salt thereof, or sodium bicarbonate, these ingredients are not included in the carrier.

The tablets according to the present invention will be described in more detail below.

In one embodiment, the food composition provided by the present invention is a tablet. The tablet provided by the present invention may contain citric acid or a salt thereof, or sodium bicarbonate, more specifically, for example, an active ingredient such as potassium citrate or a hydrate thereof; sodium citrate or a hydrate thereof; a mixture of potassium citrate monohydrate and sodium citrate dihydrate; or sodium bicarbonate, as well as pharmaceutically acceptable and edible additives that are commonly used in the pharmaceutical field. Examples of such additives include excipients, binders, disintegrants, fluidizers, flavoring agents, lubricants, pH adjusters, surfactants, stabilizers, and fragrances.

The content of citric acid or a salt thereof, or sodium bicarbonate in the tablet of the food composition provided by the present invention may be in a range of 10 to 95% by weight, preferably in a range of 30 to 90% by weight, and more preferably in a range of 60 to 85% by weight relative to the tablet.

Examples of excipients that can be used in the tablet provided by the present invention include sugars such as lactose (e.g., lactose hydrate and anhydrous lactose), glucose, sucrose, fructose, and maltose; sugar alcohols such as erythritol, sorbitol, maltitol, xylitol, and D-mannitol; starch (e.g., corn starch, potato starch, rice starch, and wheat starch), crystalline cellulose, magnesium aluminometasilicate, anhydrous calcium phosphate, precipitated calcium carbonate, calcium silicate, calcium lactate, and ethyl cellulose. Particularly, crystalline cellulose is preferable.

The content of the excipient in the tablet provided by the present invention may be in a rage of 1 to 95% by weight, preferably in a rage of 1 to 80% by weight, more preferably in a rage of 3 to 80% by weight, and still more preferably in a rage of 3 to 20% by weight relative to the tablet.

Examples of binders that can be used in the tablet provided by the present invention include hydroxypropylcellulose, hydroxypropylmethylcellulose, polyvinylpyrrolidone, dextrin, methylcellulose, polyvinyl alcohol, sodium alginate, aminoalkyl methacrylate copolymer, polyethylene glycol, pregelatinized starch (e.g., partially pregelatinized starch), agar, and gelatin. Particularly, hydroxypropyl cellulose is preferable.

The content of the binder in the tablet provided by the present invention may be in a rage of 0.1 to 30% by weight, preferably in a rage of 0.1 to 10% by weight, and more preferably in a rage o 0.3 to 3% by weight relative to the tablet.

Examples of disintegrants that can be used in the tablet provided by the present invention include croscarmellose sodium, carmellose calcium, sodium carboxymethyl starch, low substituted hydroxypropylcellulose, crospovidone, starch (e.g., wheat starch, corn starch, or partially pregelatinized starch), and carmellose. Particularly, partially pregelatinized starch is preferable.

The content of the disintegrant in the tablet provided by the present invention may be in a rage of 0.3 to 20% by weight, preferably in a rage of 1 to 10% by weight, and more preferably in a rage of 3 to 10% by weight relative to the tablet.

Examples of fluidizing agents that can be used in the tablet provided by the present invention include light anhydrous silicic acid, talc, and magnesium aluminometasilicate.

The content of the fluidizing agent in the tablet provided by the present invention may be in a range of 0.03 to 3% by weight, preferably in a range of 0.1 to 3% by weight, and more preferably in a range of 0.3 to 3% by weight relative to the tablet.

Examples of flavoring agents that can be used in the tablet provided by the present invention include acidulants such as malic acid, acetic acid, tartaric acid, fumaric acid, ascorbic acid (provided that the flavoring agents do not include citric acid or a salt thereof, or sodium bicarbonate, i.e., the active ingredient of the present invention), and sweeteners such as sodium saccharin, dipotassium glycyrrhizinate, aspartame (registered trademark), stevia, thaumatin, and sucralose.

The content of the flavoring agent in the tablet provided by the present invention may be in a range of 0.03 to 3% by weight, preferably in a range of 0.1 to 3% by weight, and more preferably in a range of 0.3 to 3% by weight relative to the tablet.

Examples of lubricants that can be used in the tablet provided by the present invention include magnesium stearate, calcium stearate, talc, light anhydrous silicic acid, sucrose fatty acid ester, carnauba wax, macrogol, and sodium stearyl fumarate. Particularly, magnesium stearate is preferable.

The content of the lubricant in the tablet provided by the present invention may be in a range of 0.1 to 30% by weight, preferably in a range of 0.3 to 10% by weight, more preferably in a range of 1 to 3% by weight relative to the tablet.

Examples of pH adjusters that can be used in the tablet provided by the present invention include phosphates (e.g., sodium dihydrogen phosphate and potassium dihydrogen phosphate), carbonates (e.g., magnesium carbonate and sodium carbonate), tartrates, fumarates, acetates, and amino acid salts (provided that the pH adjusters do not include citric acid or a salt thereof, or sodium bicarbonate, i.e., the active ingredient of the present invention).

The content of the pH adjuster in the tablet provided by the present invention may be in a range of 0.1 to 30% by weight, preferably in a range of 0.3 to 10% by weight, and more preferably in a range of 1 to 5% by weight relative to the tablet.

Examples of surfactants that can be used in the tablets provided by the present invention include sodium lauryl sulfate, polysorbate, sucrose fatty acid ester, polyoxyethylene hydrogenated castor oil, polyoxyl stearate, macrogol, and poloxamer.

The content of the surfactant in the tablet provided by the present invention may be in a range of 0.01 to 3% by weight, preferably in a range of 0.03 to 1% by weight, and more preferably in a range of 0.03 to 0.5% by weight relative to the tablet.

Examples of stabilizers that can be used in the tablet provided by the present invention include malic acid, acetic acid, tartaric acid, maleic acid, ascorbic acid, sodium edetate, tocopherol (provided that the stabilizers do not include citric acid or a salt thereof, or sodium bicarbonate, i.e., the active ingredient of the present invention).

The content of the stabilizer in the tablet provided by the present invention may be in a range of 0.01 to 30% by weight, preferably in a range of 0.1 to 30% by weight, and more preferably in a range of 1 to 20% by weight relative to the tablet.

In one embodiment, when the active ingredient of the food composition (e.g., tablet) provided by the present invention is an alkali metal salt of citric acid (e.g., potassium citrate or a hydrate thereof (e.g., potassium citrate monohydrate); sodium citrate or a hydrate thereof (e.g., sodium citrate dihydrate); a mixture of potassium citrate or a hydrate thereof and sodium citrate or a hydrate thereof; or a mixture of potassium citrate monohydrate and sodium citrate dihydrate, the food composition provided by the present invention (e.g., tablet) may contain anhydrous citric acid as a stabilizer.

Examples of fragrances that can be used in the tablet provided by the present invention include citrus fragrances such as lemon, orange, grapefruit, peppermint, spearmint, and menthol, and these fragrances may be contained in an appropriate amount per tablet (for example, in a range of 0.01 to 1% by weight, and more preferably in a range of 0.01 to 0.1% by weight per tablet).

In the tablet provided by the present invention, the total content of the active ingredient which is citric acid or a salt thereof, or sodium bicarbonate and an edible additive does not exceed 100% by weight relative to the tablet.

The tablet provided by the present invention may be an uncoated tablet containing the above-described ingredients that is not subjected to coating treatment, or may be a film-coated tablet that is subjected to coating treatment.

The content of the coating layer can be appropriately set by those skilled in the art, and may be, for example, in a range of 0.1 to 10% by weight relative to the uncoated tablet. The coating layer may appropriately contain a plasticizer, a colorant, a brightening agent, and the like, in addition to the coating base.

Examples of coating bases that can be used in the tablet provided by the present invention include hydroxypropylcellulose, hydroxypropylmethylcellulose, ethyl cellulose, cellulose acetate phthalate, methacrylic acid copolymer, and polyvinylpyrrolidone. Particularly, hydroxypropylmethylcellulose is preferable. The content of the coating base in the tablet provided by the present invention may be in a range of 0.01 to 10% by weight, and preferably in a range of 0.3 to 3% by weight relative to the tablet.

Examples of coating plasticizers that can be used in the tablet provided by the present invention include triethyl citrate, medium chain triglyceride, triacetin, glycerin, propylene glycol, and polyethylene glycol (e.g., macrogol 6000). Particularly, macrogol 6000 is preferable. The content of the coating plasticizer in the tablet provided by the present invention may be in a range of 0.01 to 1% by weight, and preferably in a range of 0.03 to 3% by weight relative to the tablet.

Examples of coating colorants that can be used in the tablet provided by the present invention include titanium oxide, yellow iron sesquioxide, iron sesquioxide, black iron oxide, FD & C BLUE No. 2, and FD & C BLUE 2 aluminum lake. The content of the coating colorant in the tablet provided by the present invention may be in a range of 0.01 to 1% by weight, and preferably in a range of 0.03 to 3% by weight relative to the tablet.

Examples of coating brighteners that can be used in the tablet provided by the present invention include carnauba wax. The content of the coating brightener in the tablet provided by the present invention may be in a range of 0.0001 to 0.1% by weight, and preferably in a range of 0.001 to 0.01% by weight relative to the tablet.

The formulation of the food composition provided by the present invention can be produced by applying a method known in the pharmaceutical field. For example, in the case of making tablets, the production method includes: mixing an active ingredient; citric acid or a salt thereof, or sodium bicarbonate (more specifically, for example, potassium citrate or a hydrate thereof; sodium citrate or a hydrate thereof; a mixture of potassium citrate monohydrate and sodium citrate dihydrate; or sodium bicarbonate) with an additive; granulating the mixture; and tableting and/or coating the granulated product.

The mixing step may include mixing the active ingredient with an additive such as an excipient, a stabilizer, a disintegrant and/or a binder. Further, the step may include mixing the mixture containing the active ingredient and the additive with a lubricant, a flavoring agent and/or a fragrance before the tableting step. Mixing can be performed using a V-type mixer, a W-type mixer, a container mixer, a tumbler mixer, a stirring mixer, or the like.

The granulation step can be performed by a granulation method known per se in the pharmaceutical field. Examples of granulation methods include a dry granulation method, a wet granulation method, and a fluidized-bed granulation method.

In one embodiment, the mixture obtained in the mixing step and the granulated product obtained in the granulation step are appropriately pulverized and/or sieved to form a mixture or granulated product having a desired particle size. The pulverization can be performed by a pulverizer known in the pharmaceutical field such as a ball mill, a jet mill, or a hammer mill. The sieving can be performed using a 16 mesh sieve (opening of 1000 µm) to 32 mesh sieve (opening of 500 µm) or the like.

The tableting step can be performed by a tableting method known per se in the pharmaceutical field. Examples of tableting methods include a direct compression tableting method, a dry tableting method, a wet tableting method, and an external lubrication tableting method.

For example, in the pharmaceutical field such as a single punch tableting machine or a rotary tableting machine, a tableting machine known per se can be used to tablet the mixture or granulated product obtained in the above step. When the single punch tableting machine, the rotary tableting machine, or the like is used, a tableting pressure of 1 kN to 30 kN can be adopted.

The coating step can be performed by a method known per se in the pharmaceutical field. For example, the step can be performed by spray-coating the outside of the uncoated tablet with a coating liquid containing a coating base and a plasticizer, a colorant, a brightening agent, and the like as appropriate.

In one embodiment, the tablet provided by the present invention can be produced by mixing the active ingredient with an excipient (e.g., lactose, D-mannitol, crystalline cellulose and/or glucose), a binder (e.g., hydroxypropylcellulose (HPC)), gelatin and/or polyvinylpyrrolidone (PVP)), a stabilizer, a disintegrant

(e.g., starch (e.g., partially pregelatinized starch) and/or carboxymethylcellulose calcium (CMC-Ca)) and a lubricant (e.g., magnesium stearate) and tableting the mixture to form an uncoated tablet; and forming a coating layer containing a coating base (e.g., hydroxypropylcellulose, hydroxypropylmethylcellulose and/or PVP), a plasticizer (e.g., triethyl citrate and/or macrogol 6000), a colorant (e.g., iron sesquioxide and/or titanium oxide), and a brightening agent (e.g., carnauba wax) on the outside of the uncoated tablet.

In one embodiment, the hardness of the resulting tablet may be in a range of 10 to 200 N, and preferably in a range of 30 to 150 N.

The amount of the active ingredient in the food composition provided by the present invention can be appropriately set. In one embodiment, the formulation of the food composition is an active ingredient, its content or dosage form that is distinguishable from a pharmaceutical formulation approved as a pharmaceutical product for improving acidic urine in gout or hyperuricemia.

When the food composition provided by the present invention is not formulated and is provided in the form of a normal food or drink, it can be appropriately produced by those skilled in the art depending on the type of the food, and can be produced, for example, by blending a food material with at least one substance selected from the group consisting of citric acid and a salt thereof (e.g., citric acid, potassium citrate or a hydrate thereof and/or sodium citrate or a hydrate thereof), or sodium bicarbonate.

Examples of forms of the food or drink include liquid or milky or pasty foods such as beverage, soy sauce, milk, yogurt, and fermented soybean paste; semi-solid foods such as jelly and gummy candy; solid foods such as candy, gum, soybean curd, and supplement; and powdered foods.

Examples of beverages include fruit juice and fruit beverages, coffee beverages, oolong tea beverages, green tea beverages, black tea beverages, barley tea beverages, vegetable beverages, soft drinks such as carbonated beverages, fruit extract-containing beverages, vegetable extract-containing juices, flavored water, sports drinks, and diet drinks.

To beverages, additives such as antioxidants, fragrances, various esters, organic acids, organic acid salts, inorganic acids, inorganic acid salts, inorganic salts, pigments, emulsifiers, preservatives, seasoning agents, sweeteners, acidulants, fruit juice extracts, vegetable extracts, flower honey extracts, pH adjusters, and quality stabilizers can be added singly or in combination.

The ingestion dose of citric acid or a salt thereof, or sodium bicarbonate as an active ingredient is appropriately determined depending on the type of the active ingredient, the method of ingestion, the age, weight, sex, and symptoms of the subject for ingestion, susceptibility to the active ingredient, and the like. The timing and dose of ingestion may be adjusted according to the type of symptom and the state of improvement or suppression thereof.

In one embodiment, the food composition provided by the present invention is in a unit package form per serving, and may contain at least one substance selected from the group consisting of citric acid and a salt in an amount of 200 mg or more, 300 mg or more, 400 mg or more, 500 mg or more, for example, in an amount of 200 mg or more and 5 g or less, 300 mg or more and 5 g or less, 400 mg or more and 5 g or less, 500 mg or more and 5 g or less, 200 mg or more and 3 g or less, 300 mg or more and 3 g or less, 400 mg or more and 3 g or less, 500 mg or more and 3 g or less, 200 mg or more and 2 g or less, 300 mg or more and 2 g or less, 400 mg or more and 2 g or less, 500 mg or more and 2 g or less, 200 mg or more and 1 g or less, 300 mg or more and 1 g or less, 400 mg or more and 1 g or less, or 500 mg or more and 1 g or less. Such a unit package form may be ingested once per day, twice per day or three times per day.

In one embodiment, the unit package form of the food composition provided by the present invention includes 1000 to 1600 mg of potassium citrate or a hydrate thereof, 1000 to 1600 mg of sodium citrate or a hydrate thereof, and 300 to 600 mg of citric acid (e.g., anhydrous citric acid). For example, such a unit package may be ingested twice per day (e.g., after breakfast and before sleep).

In one embodiment, the unit package form of the food composition provided by the present invention includes 200 to 300 mg of potassium citrate or a hydrate thereof, 200 to 300 mg of sodium citrate or a hydrate thereof, and 50 to 100 mg of citric acid (e.g., anhydrous citric acid). For example, such a unit package form may be ingested in two units at one time three times per day (e.g., after breakfast and before sleep).

In one embodiment, 1 to 10 g, 2 to 7 g, or 3 to 6 g of citric acid or a salt thereof (e.g., a mixture of citric acid, sodium citrate, and potassium citrate) may be ingested once a day by ingesting the food composition provided by the present invention.

In one embodiment, for example, in the case of foods for specified health use, nutritional supplements, foods with functional claims or foods for hospital patients, potassium citrate monohydrate and sodium citrate dihydrate may be contained in a total amount of 1/3 of 1 to 3 g per serving, or sodium bicarbonate may be contained in an amount of 1/3 of 1 to 6 g per serving. When foods for specified health use, nutritional supplements, foods with functional claims, foods for hospital patients or supplements are provided as tablets, for example, 70 to 80% by weight of citric acid or a salt thereof or sodium bicarbonate may be contained in a tablet (300 mg to 600 mg) .

The ingestion period of the food composition provided by the present invention is not particularly limited, and may be, for example, 1 day or more, 2 days or more, 3 days or more, 1 week or more, 2 weeks or more, or 4 weeks or more.

The food composition provided by the present invention can be applied to a subject in need of improvement in quality of sleep in one embodiment.

In one embodiment, the subject in need of improvement in quality of sleep means a mammal (particularly a human) who does not have "morbid", "abnormal" or "unpleasant" symptoms, conditions or diseases in the quality of sleep; or a subject who has "morbid", "abnormal" or "unpleasant" symptoms, but does not seem to have any disease or disorder, i.e., a subject in a "healthy" condition. The food composition provided by the present invention can be applied to maintain or enhance "healthy", "normal" or "comfortable" conditions, or make the "comfortable" condition more comfortable, or improve the "morbid", "abnormal" or "unpleasant" symptoms. Hence, in one embodiment, the "improvement in quality of sleep" by the food composition provided by the present invention is a concept including "improvement in quality of sleep".

In one embodiment, the food composition provided by the present invention can be applied to "an individual who is concerned about the quality of sleep (healthy individual)", "an individual who has difficulty falling asleep (healthy individual)", "an individual who wakes up in the middle of the night (healthy individual)", "an individual who wants to sleep soundly until morning (healthy individual)", "an individual who is in a daze in the morning (healthy individual)", "an individual who is concerned about urge to urinate at night or urination at night (e.g., the frequency of nocturnal urination is high) (healthy individual)", "an individual who is concerned about sleepiness during the day (healthy individual)" or "an individual who does not awake up well (healthy individual)", in order to improve the quality of sleep. In this case, the above-described ingestion dose of the food composition provided by the present invention may be taken together with a normal meal, before or after the normal meal, and it can be taken before bedtime, for example, up to 3 hours, more preferably 0.5 hours to 2 hours before the scheduled bedtime.

In one embodiment, the food composition provided by the present invention may be ingested before or after a normal meal, or after breakfast and before bedtime (e.g., 30 minutes before bedtime).

In one embodiment, the food composition provided by the present invention is ingested by a healthy individual, which can exert beneficial effects (e.g., an effect of reducing nocturnal urine production, an effect of reducing the frequency of nocturnal urination, and an effect of suppressing awakening after sleep onset, an effect of improving the quality of sleep (e.g., an effect of improving the quality of sleep due to a reduced urge to urinate at night or a decrease in the frequency of nocturnal urination).

In one embodiment, the food composition provided by the present invention does not affect the daily urine output.

In one embodiment, the food composition provided by the present invention does not affect the frequency of urination per day.

In one embodiment, the food composition provided by the present invention is ingested by an elderly individual (e.g., 60 years old or older, 65 years old or older, 70 years old or older, 75 years old or older, or 65 years old or older and under 75).

In one embodiment, the food composition provided by the present invention is administered to an individual 40 years old or older, 50 years old or older, or 40 years old or older and under 60.

In one embodiment, the food composition provided by the present invention is ingested by a young individual (e.g., an infant (e.g., a child 3 years old or older and under 6), a child (e.g., a child 6 years old or older), a child aged between 6 and 12, and a child aged between 6 and 15) .

In one embodiment, the food composition provided by the present invention is ingested by "an age-conscious individual".

In one embodiment, the effective amount of the food composition provided by the present invention is administered to a subject in need of an increase in blood ornithine level, blood serotonin level, blood taurine level, blood cystine level, or blood nicotine amide level.

In one embodiment, a subject in need of an increase in blood ornithine level, blood serotonin level or blood taurine level may be the same as the subject in need of improvement in quality of sleep.

Therefore, embodiments of the food composition according to the present invention include the following:
<1-1> A food composition for improving quality of sleep in mammals (particularly humans), including, as an active ingredient, citric acid or a salt thereof, or sodium bicarbonate;
<1-2> A method for improving quality of sleep, including allowing a subject in need of improvement in quality of sleep to ingest an effective amount of a food composition containing citric acid or a salt thereof, or sodium bicarbonate; and
<1-3> Use of citric acid or a salt thereof, or sodium bicarbonate for producing a food composition for improving quality of sleep.

It is preferable that a package, container, or instruction of the food composition according to the present invention indicates an effect of improving the quality of sleep.

Another aspect of the embodiments of the food composition according to the present invention includes the following:
<2-1> A food composition for increasing a blood ornithine level, a blood serotonin level, a blood taurine level, a blood cystine level, or a blood nicotine amide level in mammals (particularly humans), including, as an active ingredient, citric acid or a salt thereof or sodium bicarbonate;
<2-2> A method for increasing a blood ornithine level, a blood serotonin level, a blood taurine level, a blood cystine level, or a blood nicotine amide level, including allowing a subject in need of an increase in blood ornithine level, blood serotonin level, blood taurine level, blood cystine level, or blood nicotine amide level to ingest an effective amount of a food composition containing citric acid or a salt thereof, or sodium bicarbonate; and
<2-3> Use of citric acid or a salt thereof, or sodium bicarbonate for producing a food composition for increasing a blood ornithine level, a blood serotonin level, a blood taurine level, a blood cystine level, or a blood nicotine amide level.

Another aspect of the embodiments of the food composition according to the present invention includes the following:
<3-1> A food composition for suppressing awakening after sleep onset, including, as an active ingredient, citric acid or a salt thereof, or sodium bicarbonate;
<3-2> A method for suppressing awakening after sleep onset including allowing a subject in need of suppression in awakening after sleep onset to ingest an effective amount of a food composition containing citric acid or a salt thereof, or sodium bicarbonate; and
<3-3> Use of citric acid or a salt thereof, or sodium bicarbonate for producing a food composition for suppressing awakening after sleep onset.

Another aspect of the embodiments of the food composition according to the present invention includes the following:
<4-1> A food composition for improving quality of sleep (e.g., for improving the quality of sleep due to a reduced urge to urinate at night or a decrease in the frequency of nocturnal urination), which includes, as an active ingredient, citric acid or a salt thereof or sodium bicarbonate;
<4-2> A method for improving quality of sleep, including allowing a subject in need of improvement in quality of sleep (e.g., improvement in the quality of sleep due to a reduced urge to urinate at night or a decrease in the frequency of nocturnal urination) to ingest an effective amount of a food composition containing citric acid or a salt thereof, or sodium bicarbonate (e.g., a method for improving the quality of sleep due to a reduced urge to urinate at night or a decrease in the frequency of nocturnal urination); and
<4-3> Use of citric acid or a salt thereof, or sodium bicarbonate for producing a food composition for improving quality of sleep (e.g., a food composition for improving the quality of sleep due to a reduced urge to urinate at night or a decrease in the frequency of nocturnal urination).

Another aspect of the embodiments of the food composition according to the present invention includes the following:
<5-1> The food composition, method, or use according to any one of <1-1> to <1-3>, <2-1> to <2-3>, <3-1> to <3-3>, and <4-1> to <4-3>, wherein the food composition is ingested by an elderly individual or a young individual; and
<5-2> The food composition, method, or use according to any one of <1-1> to <1-3>, <2-1> to <2-3>, <3-1> to <3-3>, <4-1> to <4-3>, and <5-1>, wherein the food composition is ingested by an age-conscious individual.

The blood ornithine level, blood serotonin level, blood taurine level, blood cystine level, and blood nicotine amide level can be measured by a known HPLC method, enzyme method, enzyme immunoassay method, or the like.

Another aspect of the embodiments of the food composition according to the present invention is a food composition for improving the quality of sleep in patients suffering from kidney disease (in the present specification, sometimes referred to as "patients with kidney disease").

Another aspect of the embodiments of the food composition according to the present invention is a food composition for increasing a blood ornithine level, a blood serotonin level, a blood taurine level, a blood cystine level, or a blood nicotine amide level in patients with kidney disease.

In the present specification, the term "kidney disease" includes acute kidney disease and chronic kidney disease unless otherwise specified.

Examples of acute kidney disease include acute kidney disease due to drugs (e.g., non-steroidal antiinflammatory drugs, angiotensin-converting enzyme inhibitors, angiotensin II receptor antagonists, aminoglycoside antibiotics, new quinolone antibacterial agents, iodine contrast agents, platinum-containing drugs such as cisplatin) and acute kidney disease due to renal ischemia.

Chronic kidney disease (CKD) is a concept that includes chronic kidney disease following a chronic course regardless of the underlying disease, and the concept includes the presence of decreased renal function represented by glomerular filtration rate (GFR), or all pathological conditions of the findings suggesting kidney damage which persists chronically (3 months or more).

### 2. Pharmaceutical composition

The pharmaceutical composition provided by the present invention may include, as an active ingredient, citric acid or a salt thereof, or sodium bicarbonate. Citric acid or a salt thereof or sodium bicarbonate included in the pharmaceutical composition is synonymous with citric acid or a salt thereof or sodium bicarbonate described as an active ingredient of the food composition, and the specific examples thereof are the same as those described above.

In one embodiment, the pharmaceutical composition provided by the present invention may include, as an active ingredient, at least one substance selected from the group consisting of citric acid and a salt of citric acid.

In one embodiment, when the active ingredient of the pharmaceutical composition (e.g., tablet) provided by the present invention is an alkali metal salt of citric acid (e.g., potassium citrate or a hydrate thereof (e.g., potassium citrate monohydrate); sodium citrate or a hydrate thereof (e.g., sodium citrate dihydrate); a mixture of potassium citrate or a hydrate thereof and sodium citrate or a hydrate thereof; or a mixture of potassium citrate monohydrate and sodium citrate dihydrate, the pharmaceutical composition provided by the present invention (e.g., tablet) may contain anhydrous citric acid as a stabilizer.

In one embodiment, administration of the pharmaceutical composition provided by the present invention leads to an improvement in the quality of sleep in mammals (particularly humans). For example, comparison between the pre-administration of the pharmaceutical composition provided by the present invention and the placebo is performed, and the "improvement in quality of sleep" can be evaluated as suppression of awakening after sleep onset (e.g., decrease in frequency of awakening after sleep onset), promotion of slow-wave sleep (e.g., increase in duration of slow-wave sleep (e.g., NREM sleep stage 3), increase in percentage of duration of slow-wave sleep (e.g., NREM sleep stage 3) to duration of total sleep, increase in delta power in the first sleep cycle), suppression of early-morning awakening (e.g., decrease in frequency of awakening after sleep onset 2 hours before waking up), increase in duration of slow-wave sleep, increase in percentage of duration of slow-wave sleep to duration of total sleep, or promotion of deep sleep (e.g., increase in delta power (e.g., the delta power of the first sleep cycle)) or improvement in sound sleep feeling.

The pharmaceutical composition provided by the present invention is orally or parenterally administered to a human or another mammal, and examples of parenteral administration include intravenous administration, subcutaneous administration, intramuscular administration, intraarticular administration, and transmucosal administration, transdermal administration, nasal administration, rectal administration, intrathecal administration, intraperitoneal administration, and local administration.

The pharmaceutical composition provided by the present invention may be prepared by using citric acid or a salt thereof, or sodium bicarbonate as it is, or by mixing citric acid or a salt thereof, or sodium bicarbonate with a pharmaceutically acceptable carrier such as an excipient, a binder, a lubricant, a disintegrant, a diluent, and if necessary, other additives, and may be a formulation such as a tablet, a capsule, a suspension, an injection, or a suppository.

The excipient, binder, lubricant, disintegrant, diluent, and other additives described in the food composition can be used for the excipient, binder, lubricant, disintegrant, diluent, and other additives as described above. Further, the food composition provided by the present invention can be formulated into a tablet, a capsule, a suppository, or the like suitable for oral ingestion, whereas the pharmaceutical composition provided by the present invention can be formulated into a dosage form suitable for parenteral administration such as an injection or a suppository, in addition to a dosage form suitable for oral ingestion like the food composition.

In one embodiment, the pharmaceutical composition provided by the present invention is a tablet. The content of at least one substance selected from the group consisting of citric acid and a salt of citric acid, or sodium bicarbonate in the tablet of the pharmaceutical composition provided by the present invention is in a range of 10 to 95% by weight, preferably in a range of 30 to 90% by weight, and more preferably in a range of 60 to 85% by weight relative to the tablet.

The term "ingestion" described in "1. Food composition" above can also be applied to the "pharmaceutical composition" according to the present invention. Further, regarding the "pharmaceutical composition" according to the present invention, the term "ingestion" may be replaced with "administration". Therefore, for example, the term "ingest", "allowing a subject to ingest", "ingested", or the like can be changed and replaced with "administer", "administered", or the like, depending on the context. The technology applied to the formulated food composition can also be applied to the pharmaceutical composition.

In the pharmaceutical composition provided by the present invention, the amount of citric acid or a salt thereof, or sodium bicarbonate, which is an active ingredient, can be appropriately set. For example, a formulation of a pharmaceutical composition may contain 70 to 80% by weight of at least one substance selected from the group consisting of citric acid and a salt of citric acid, or sodium bicarbonate per formulation. When the pharmaceutical composition is provided as tablets, 70 to 80% by weight of at least one substance selected from the group consisting of citric acid and a salt of citric acid, or sodium bicarbonate may be contained in a tablet (300 mg to 600 mg).

In one embodiment, the salt of citric acid is a pharmaceutically acceptable salt other than ferrous citrate and ferric citrate.

In one embodiment, among the active ingredients in the pharmaceutical composition provided by the present invention, the amount of an alkaline agent such as an alkali metal salt of citric acid or sodium bicarbonate may be set to a value in which acidic urine in gout and hyperuricemia is improved by administering the alkaline agent to a human, or a value less than the above value. For example, the amount may be set to 1 to 50% or 10 to 20% of the daily dose approved in Japan for improving acidic urine in gout and hyperuricemia (e.g., when the alkalizing agent is a citric acid formulation: two tablets each containing 231.5 mg of potassium citrate (C₆H₅K₃O₇·H₂O) and 195.0 mg of sodium citrate hydrate (C₆H₅Na₃O₇·2H₂O are orally administered three times per day, when the alkalizing agent is sodium bicarbonate: 3 to 5 g of sodium bicarbonate is orally administered per day).

In one embodiment, the pharmaceutical composition provided by the present invention is a tablet, and may contain potassium citrate monohydrate or sodium citrate dihydrate as an alkalizing agent in an amount of 10 mg to 1 g, preferably in an amount of 100 mg to 500 mg, more preferably in an amount of 400 mg to 500 mg per tablet.

In one embodiment, the pharmaceutical composition provided by the present invention is a tablet, and may contain potassium citrate monohydrate and sodium citrate dihydrate, respectively, in an amount of 10 mg to 300 mg for a total of 20 mg to 600 mg, preferably in an amount of 150 to 250 mg for a total of 400 to 500 mg, more preferably in an amount of 190 to 240 mg for a total of 400 to 450 mg per tablet.

In one embodiment, the pharmaceutical composition provided by the present invention is a tablet, and may contain sodium bicarbonate as an alkalizing agent in an amount of 10 mg to 1 g, preferably in an amount of 100 mg to 500 mg per tablet.

In one embodiment, the pharmaceutical composition provided by the present invention is a tablet, and may contain 231.5 mg of potassium citrate monohydrate and 195.0 mg of sodium citrate dihydrate as active ingredients, and may contain anhydrous citric acid, crystalline cellulose, partially pregelatinized starch, hydroxypropylcellulose, magnesium stearate, hypromellose, macrogol 6000, titanium oxide, and carnauba wax as additives.

In one embodiment, a tablet containing 231.5 mg of potassium citrate monohydrate and 195.0 mg of sodium citrate dihydrate may be used as one dosage unit.

In one embodiment, a tablet containing 231.5 mg of potassium citrate monohydrate, 195.0 mg of sodium citrate dihydrate, and 72.5 mg of anhydrous citric acid may be used as one dosage unit.

In the present specification, the "dosage unit" represents a unit of the formulation, and the "one dosage unit" represents the minimum unit of the formulation. Thus, for example, in the case of tablets, the dosage unit is each tablet and one dosage unit represents one tablet. In the case of an injection, the dosage unit is an injection placed in a sealed container such as an ampoule or a vial, and one administration unit represents an injection in a hermetically sealed container such as an ampoule or a vial.

When the pharmaceutical composition provided by the present invention is administered to a human or another mammal, one or more of the above-described dosage units may be administered at a time, and the one dosage unit may be administered in divided doses.

The administered dose of an active ingredient, which is citric acid or a salt thereof, or sodium bicarbonate, is appropriately determined according to the type of the active ingredient, the administration method, the age, weight, sex, and symptoms of the subject to be administered, susceptibility to drugs, and the like. The administered dose may be adjusted according to the situation of symptom improvement.

In one embodiment, when the active ingredient; a mixture of potassium citrate monohydrate and sodium citrate dihydrate or sodium bicarbonate is orally administered to a human, half of the daily dose approved in Japan for improving acidic urine in gout and hyperuricemia (e.g., when the active ingredient is a citric acid formulation: two tablets each containing 231.5 mg of potassium citrate (C₆H₅K₃O₇·H₂O) and 195.0 mg of sodium citrate hydrate (C₆H₅Na₃O₇·2H₂O) are orally administered three times per day, when the active ingredient is sodium bicarbonate: 3 to 5 g of sodium bicarbonate is orally administered per day) may be used as the daily dose.

In one embodiment, when the active ingredient; a mixture of potassium citrate monohydrate and sodium citrate dihydrate or sodium bicarbonate is orally administered to a human, the daily dose approved in Japan for improving acidic urine in gout and hyperuricemia (e.g., when the active ingredient is a citric acid formulation: two tablets each containing 231.5 mg of potassium citrate (C₆H₅K₃O₇·H₂O) and 195.0 mg of sodium citrate hydrate (C₆H₅Na₃O₇·2H₂O) are orally administered three times per day, when the active ingredient is sodium bicarbonate: 3 to 5 g of sodium bicarbonate is orally administered per day) may be used as the daily dose.

In one embodiment, when the active ingredient; a mixture of potassium citrate monohydrate and sodium citrate dihydrate or sodium bicarbonate is orally administered to a human, half of the daily dose approved in Japan for improving acidic urine in gout and hyperuricemia (e.g., when the active ingredient is a citric acid formulation: two tablets each containing 231.5 mg of potassium citrate (C₆H₅K₃O₇·H₂O) and 195.0 mg of sodium citrate hydrate (C₆H₅Na₃O₇·2H₂O) are orally administered three times per day, when the active ingredient is sodium bicarbonate: 3 to 5 g of sodium bicarbonate is orally administered per day) is used as the daily dose, the administration is started, and then the dose may be increased to the daily dose to improve acidic urine in gout and hyperuricemia.

In one embodiment, when the active ingredient; a mixture of potassium citrate monohydrate and sodium citrate dihydrate is orally administered to a human, each of potassium citrate monohydrate and sodium citrate dihydrate may be administered in a dose of 0.1 to 5 g/day for a total of 0.2 to 10 g/day, 0.1 to 3 g/day for a total of 0.2 to 6 g/day, 0.5 to 3 g/day for a total of 1 to 6 g/day, preferably 0.5 to 1.5 g/day for a total of 1 to 3 g/day, 1 to 1.5 g/day for a total of 2 to 3 g/day, or 0.5 to 1 g/day for a total of 1 to 2 g/day, and may be administered daily in 1 to 5 divided doses, preferably 3 divided doses.

In one embodiment, when the active ingredient; potassium citrate monohydrate or sodium citrate dihydrate is orally administered to a human, it may be administered in a dose of 1 to 10 g/day, 1 to 6 g/day, 2 to 5.5 g/day, 1 to 3 g/day, 2 to 3 g/day, or 1 to 1.5 g/day, and may be administered daily in 1 to 5 divided doses, preferably 3 divided doses.

In one embodiment, when the active ingredient; sodium bicarbonate is orally administered to a human, it may be administered in a dose of 1 to 6g/day, preferably 1 to 3 g/day, or 3 to 5g/day, and may be administered daily in 1 to 5 divided doses, preferably 3 divided doses.

In one embodiment, the active ingredient may be administered sequentially. Particularly, in order to improve the quality of sleep, the active ingredient is administered, for example, for 2 days, 3 days, 5 days, 1 week, 2 weeks, 3 weeks, 6 weeks, 8 weeks, 10 weeks, 12 weeks, 24 weeks, 40 weeks, 60 weeks, 80 weeks, 100 weeks, 120 weeks, 1 week or more, 2 weeks or more, 3 weeks or more, 6 weeks or more, 8 weeks or more, 10 weeks or more, 12 weeks or more, 24 weeks or more, 40 weeks or more, 60 weeks or more, 80 weeks or more, 100 weeks or more, 120 weeks or more, 6 weeks to 24 weeks, 12 weeks to 24 weeks, 6 weeks to 30 weeks, 12 weeks to 30 weeks, 6 weeks to 40 weeks, 12 weeks to 40 weeks, 6 weeks to 60 weeks, 12 weeks to 60 weeks, 6 weeks to 80 weeks, 12 weeks to 80 weeks, 6 weeks to 100 weeks, 12 weeks to 100, 6 weeks to 120 weeks, or 12 weeks to 120 weeks.

In one embodiment, the active ingredient may be administered sequentially. The active ingredient is administered, for example, for 2 days, 3 days, 5 days, 1 week, 2 weeks, particularly when used for improving the quality of sleep.

In one embodiment, an effective amount of the pharmaceutical composition provided by the present invention is administered to a subject (e.g., a human or another mammal) who needs to improve the quality of sleep.

In one embodiment, an effective amount of the pharmaceutical composition provided by the present invention is administered to a patient who needs the treatment of insomnia (e.g., awakening after sleep onset, early-morning awakening, or deep sleep disorder).

In one embodiment, the effective amount of the pharmaceutical composition provided by the present invention is administered to a subject in need of an increase in blood ornithine level, blood serotonin level, blood taurine level, blood cystine level, or blood nicotine amide level.

In one embodiment, a subject in need of an increase in blood ornithine level, blood serotonin level or blood taurine level may be the same as the subject in need of improvement in quality of sleep.

Since nocturnal polyuria and increased frequency of nocturnal urination are commonly seen in elderly individuals, in one embodiment, the pharmaceutical composition provided by the present invention is administered to an elderly individual (e.g., 60 years old or older, 65 years old or older, 70 years old or older, 75 years old or older, 65 years old or older and under 75).

In one embodiment, the pharmaceutical composition provided by the present invention is administered to a human 40 years old or older, 50 years old or older, or 40 years old or older and under 60.

Further, in chronic kidney disease, urination disorders such as polyuria and nocturnal polyuria is observed from a relatively early stage (e.g., a human whose CKD stage is G3a or G3b). Accordingly, in one embodiment, the pharmaceutical composition provided by the present invention is administered to a patient with chronic kidney disease (e.g., a human whose CKD stage is G3a or G3b).

Furthermore, nocturnal enuresis is a problem in young individuals. Accordingly, in one embodiment, the pharmaceutical composition provided by the present invention is administered to a young individual (e.g., an infant (e.g., a child 3 years old or older and under 6), a child (e.g., a child 6 years old or older), a child aged between 6 and 12, and a child aged between 6 and 15).

In one embodiment, the subject of administration of the pharmaceutical composition provided by the present invention does not suffer from gout.

In one embodiment, the subject of administration of the pharmaceutical composition provided by the present invention does not suffer from hyperuricemia.

In one embodiment, the subject of administration of the pharmaceutical composition provided by the present invention is not a subject (e.g., a human) in need of improvement of acidic urine in gout and hyperuricemia.

In one embodiment, the subject of administration of the pharmaceutical composition provided by the present invention is not a subject (e.g., a human) in need of improvement of acidosis.

In one embodiment, the pharmaceutical composition provided by the present invention is not used in combination with a uric acid lowering agent (e.g., a uric acid excretion promoter or a uric acid production inhibitor).

Examples of other embodiments of the present invention include following:
(1-1) A method of improving quality of sleep, including administering an effective amount of a pharmaceutical composition containing citric acid or a salt thereof, or sodium bicarbonate, to a subject in need of improvement in quality of sleep;
(1-2) A method for increasing a blood ornithine level, a blood serotonin level, a blood taurine level, a blood cystine level, and a blood nicotine amide level, including administering an effective amount of a pharmaceutical composition containing citric acid or a salt thereof, or sodium bicarbonate, to a subject in need of an increase in blood ornithine level, blood serotonin level, blood taurine level, blood cystine level, or blood nicotine amide level;
(1-3) A method for treating or preventing sleep disorders associated with frequent urination (e.g., nocturia, or nocturia due to nocturnal polyuria), including administering an effective amount of a pharmaceutical composition containing citric acid or a salt thereof, or sodium bicarbonate, to a subject in need of treatment or prevention of sleep disorders associated with frequent urination (e.g., nocturia, or nocturia due to nocturnal polyuria);
(1-4) A method for suppressing awakening after sleep onset, including administering an effective amount of a pharmaceutical composition containing citric acid or a salt thereof, or sodium bicarbonate, to a subject in need of suppression in awakening after sleep onset;
(1-5) A method for improving quality of sleep, including administering an effective amount of a pharmaceutical composition containing citric acid or a salt thereof, or sodium bicarbonate, to a subject in need of improvement in quality of sleep (e.g., improvement in the quality of sleep due to a reduced urge to urinate at night or a decrease in the frequency of nocturnal urination) (e.g., a method for improving the quality of sleep due to a reduced urge to urinate at night or a decrease in the frequency of nocturnal urination);
(1-6) The method according to any one of (1-1) to (1-5), wherein the subject is a patient suffering from kidney disease;

(2-1) A pharmaceutical composition including citric acid or a salt thereof, or sodium bicarbonate for use in improving quality of sleep;
(2-2) A pharmaceutical composition including citric acid or a salt thereof, or sodium bicarbonate for use in improving quality of sleep in patients with kidney disease;
(2-3) A pharmaceutical composition including citric acid or a salt thereof, or sodium bicarbonate for use in increasing a blood ornithine level, a blood serotonin level, a blood taurine level, a blood cystine level, and a blood nicotine amide level;
(2-4) A pharmaceutical composition including citric acid or a salt thereof, or sodium bicarbonate for use in increasing a blood ornithine level, a blood serotonin level, a blood taurine level, a blood cystine level, and a blood nicotine amide level in patients with kidney disease;
(2-5) A pharmaceutical composition including citric acid or a salt thereof, or sodium bicarbonate for use in treating or preventing sleep disorders associated with frequent urination (e.g., nocturia, or nocturia due to nocturnal polyuria);
(2-6) A pharmaceutical composition including citric acid or a salt thereof, or sodium bicarbonate for use in treating or preventing sleep disorders associated with frequent urination (e.g., nocturia, or nocturia due to nocturnal polyuria) in patients with kidney disease;
(2-7) A pharmaceutical composition including citric acid or a salt thereof, or sodium bicarbonate for use in suppressing awakening after sleep onset;
(2-8) A pharmaceutical composition including citric acid or a salt thereof, or sodium bicarbonate for use in suppressing awakening after sleep onset in patients with kidney disease;
(2-9) A pharmaceutical composition including citric acid or a salt thereof, or sodium bicarbonate for use in improving quality of sleep (e.g., improving the quality of sleep due to a reduced urge to urinate at night or a decrease in the frequency of nocturnal urination);
(2-10) A pharmaceutical composition including citric acid or a salt thereof, or sodium bicarbonate for use in improving quality of sleep in patients with kidney disease (e.g., improving the quality of sleep due to a reduced urge to urinate at night or a decrease in the frequency of nocturnal urination);

(3-1) Use of citric acid or a salt thereof, or sodium bicarbonate for producing a pharmaceutical composition for improving quality of sleep;
(3-2) Use of citric acid or a salt thereof, or sodium bicarbonate for producing a pharmaceutical composition for improving quality of sleep in patients with kidney disease;
(3-3) Use of citric acid or a salt thereof, or sodium bicarbonate for producing a pharmaceutical composition for increasing a blood ornithine level, a blood serotonin level, a blood taurine level, a blood cystine level, and a blood nicotine amide level;
(3-4) Use of citric acid or a salt thereof, or sodium bicarbonate for producing a pharmaceutical composition for increasing a blood ornithine level, a blood serotonin level, a blood taurine level, a blood cystine level, and a blood nicotine amide level in patients with kidney disease;
(3-5) Use of citric acid or a salt thereof, or sodium bicarbonate for producing a pharmaceutical composition for treating or preventing sleep disorders associated with frequent urination (e.g., nocturia, or nocturia due to nocturnal polyuria);
(3-6) Use of citric acid or a salt thereof, or sodium bicarbonate for producing a pharmaceutical composition for treating or preventing sleep disorders associated with frequent urination (e.g., nocturia, or nocturia due to nocturnal polyuria) in patients with kidney disease;
(3-7) Use of citric acid or a salt thereof, or sodium bicarbonate for producing a pharmaceutical composition for suppressing awakening after sleep onset;
(3-8) Use of citric acid or a salt thereof, or sodium bicarbonate for producing a pharmaceutical composition for suppressing awakening after sleep onset in patients with kidney disease;
(3-9) Use of citric acid or a salt thereof, or sodium bicarbonate for producing a pharmaceutical composition for improving quality of sleep (e.g., a pharmaceutical composition for improving the quality of sleep due to a reduced urge to urinate at night or a decrease in the frequency of nocturnal urination);
(3-10) Use of citric acid or a salt thereof, or sodium bicarbonate for producing a pharmaceutical composition for improving quality of sleep in patients with kidney disease (e.g., a pharmaceutical composition for improving the quality of sleep due to a reduced urge to urinate at night or a decrease in the frequency of nocturnal urination);

(4-1) The method, pharmaceutical composition, or use according to any one of (1-1) to (1-6), (2-1) to (2-10), and (3-1) to (3-10), wherein the pharmaceutical composition is administered to a patient with chronic kidney disease;
(4-2) The method, pharmaceutical composition, or use according to any one of (1-1) to (1-6), (2-1) to (2-10), (3-1) to (3-10), and (4-1), wherein the content of citric acid or a salt thereof, or sodium bicarbonate in the pharmaceutical composition is an amount that improves acidic urine in gout or hyperuricemia;
(4-3) The method, pharmaceutical composition, or use according to any one of (1-1) to (1-6), (2-1) to (2-10), (3-1) to (3-10), (4-1), and (4-2), wherein the pharmaceutical composition is not administered to a patient with gout and a patient with hyperuricemia; and
(4-4) The method, pharmaceutical composition, or use according to any one of (1-1) to (1-6), (2-1) to (2-10), (3-1) to (3-10), and (4-1) to (4-3), wherein the pharmaceutical composition is administered for 1 week.

In one embodiment, the pharmaceutical composition provided by the present invention is a pharmaceutical composition including an alkali metal salt of citric acid for improving the quality of sleep. As the alkali metal salt of citric acid, each of potassium citrate monohydrate and sodium citrate dihydrate is orally administered in a dose of 0.5 to 1.5 g/day for a total of 1 to 3 g/day, and is administered daily in 1 to 5 divided doses, preferably 3 divided doses.

In one embodiment, the pharmaceutical composition provided by the present invention is a pharmaceutical composition including an alkali metal salt of citric acid for improving the quality of sleep, one dosage unit (preferably one tablet) contains, as the alkali metal salt of citric acid, 231.5 mg of potassium citrate monohydrate and 195.0 mg of sodium citrate dihydrate, and three to six dosage units are orally administered daily in 3 divided doses.

In one embodiment, the pharmaceutical composition provided by the present invention is a pharmaceutical composition including an alkali metal salt of citric acid for improving the quality of sleep in patients with kidney disease. As the alkali metal salt of citric acid, each of potassium citrate monohydrate and sodium citrate dihydrate is orally administered in a dose of 0.5 to 1.5 g/day for a total of 1 to 3 g/day, and is administered daily in 1 to 5 divided doses, preferably 3 divided doses.

In one embodiment, the pharmaceutical composition provided by the present invention is a pharmaceutical composition including an alkali metal salt of citric acid for improving the quality of sleep in patients with kidney disease, one dosage unit (preferably one tablet) contains, as an alkali metal salt of citric acid, 231.5 mg of potassium citrate monohydrate and 195.0 mg of sodium citrate dihydrate, and three to six dosage units are orally administered daily in 3 divided doses.

In one embodiment, the pharmaceutical composition provided by the present invention is a pharmaceutical composition including an alkali metal salt of citric acid for increasing a blood ornithine level, a blood serotonin level, a blood taurine level, a blood cystine level, and a blood nicotine amide level. As the alkali metal salt of citric acid, each of potassium citrate monohydrate and sodium citrate dihydrate is orally administered in a dose of 0.5 to 1.5 g/day for a total of 1 to 3 g/day, and is administered daily in 1 to 5 divided doses, preferably 3 divided doses.

In one embodiment, the pharmaceutical composition provided by the present invention is a pharmaceutical composition including an alkali metal salt of citric acid for increasing a blood ornithine level, a blood serotonin level, a blood taurine level, a blood cystine level, and a blood nicotine amide level. One dosage unit (preferably one tablet) contains, as the alkali metal salt of citric acid, 231.5 mg of potassium citrate monohydrate and 195.0 mg of sodium citrate dihydrate, and three to six dosage units are orally administered daily in 3 divided doses.

In one embodiment, the pharmaceutical composition provided by the present invention is a pharmaceutical composition including an alkali metal salt of citric acid for increasing a blood ornithine level, a blood serotonin level, a blood taurine level, a blood cystine level, and a blood nicotine amide level in patients with kidney disease. As the alkali metal salt of citric acid, each of potassium citrate monohydrate and sodium citrate dihydrate is orally administered in a dose of 0.5 to 1.5 g/day for a total of 1 to 3 g/day, and is administered daily in 1 to 5 divided doses, preferably 3 divided doses.

In one embodiment, the pharmaceutical composition provided by the present invention is a pharmaceutical composition including an alkali metal salt of citric acid for increasing a blood ornithine level, a blood serotonin level, a blood taurine level, a blood cystine level, and a blood nicotine amide level in patients with kidney disease. One dosage unit (preferably one tablet) contains, as the alkali metal salt of citric acid, 231.5 mg of potassium citrate monohydrate and 195.0 mg of sodium citrate dihydrate, and three to six dosage units are orally administered daily in 3 divided doses.

Hereinafter, the present invention will be further described with reference to Examples, but the present invention is not limited thereto.

### Examples

### Example 1

Four citric acid formulation tablets (each containing 231.5 mg of potassium citrate monohydrate, 195.0 mg of sodium citrate dihydrate, and 72.5 mg of citric acid) were mixed with 200 g of a forage, and miniature pig models with renal damage due to ligation of renal vessels (Gottingen Minipigs, male, n = 3, 20 months old, Oriental Yeast Co., Ltd., Ina MP Breeding Center) were fed with the resulting mixture three times per day for 7 days (stage I). Thereafter, a 7-day washout period was set, and the forage containing four citric acid formulation tablets was fed three times per day for 7 days (stage II). Blood samples were collected at 4 points: before administration of the citric acid formulation in stage I (1), the last day of administration (2), the last day of washout period (3), and the last day of administration in stage II (4), and plasma samples were obtained. After appropriate pretreatment, the samples were measured for metabolites by capillary electrophoresis-mass spectrometry using CE-TOFMS (Agilent Technologies, Inc.). The amount of the obtained metabolites was calculated as a relative area value, and comparison between groups was performed by Welch's t-test.

As a result, the following was confirmed.

It was confirmed that the ornithine level in stage II (4) was increased compared to that before administration of the citric acid formulation (1) (p = 0.0711) (Fig. 1).

It was confirmed that the serotonin level in stage II (4) was increased compared to that in the washout period (3) (p = 0.0511) (Fig. 2).

It was confirmed that the taurine level tended to be increased in stage I (2) compared to that before the start of the test (1), and the taurine level tended to be increased in stage II (4) compared to that in the washout period (3) (Fig. 3).

It was confirmed that the cystine level in stage II (4) was significantly increased compared to that in the washout period (3) (Fig. 4).

It was confirmed that the nicotinamide level in stage II (4) was increased compared to that before the start of the test (1) and that in the washout period (2) (Fig. 5).

### Example 2

Subjects were 25 men and women who were judged to be "suspected of insomnia" with a score of 6 to 9 on the Athens Insomnia Scale. Each of the subjects daily took a capsule containing placebo or 3 g of a salt of citric acid (citrate) (the capsule contained 1392 mg of potassium citrate monohydrate, 1170 mg of sodium citrate dihydrate, and 438 mg of citric acid) after breakfast and 30 minutes before bedtime for 2 weeks The washout period was one week. On the morning after the last day of administration, all subjects were surveyed using the St. Mary's Hospital Sleep Questionnaire (SMH). 9 of the subjects had electroencephalographs at bedtime and measured their brain waves until waking up. 23 subjects (mean age of 38.4 years) were analyzed for SMH, 8 subjects (mean age of 43.5 years) were subjected to electroencephalogram examination, and they were compared to the placebo based on the Wilcoxon signed rank sum test. As a result, the following was confirmed.

The results of the St. Mary's Hospital Sleep Questionnaire confirmed that the frequency of awakening after sleep onset in the citrate group was significantly reduced, compared to that in the placebo group (Fig. 6).

It was confirmed that the citrate group slept deeply (Fig. 7) and had a good sleep, compared to the placebo group (Fig. 8).

The electroencephalogram examination results confirmed that the frequency of awakening after sleep onset (Fig. 9) and the frequency of early-morning awakening (Fig. 10) in the citrate group were reduced, compared to the placebo group.

It was confirmed that, in the citrate group, the percentage (Fig. 11) and duration (Fig. 12) of NREM sleep stage 1 (light sleep) were reduced, and the percentage (Fig. 13) and duration (Fig. 14) of NREM sleep stage 3 (slow-wave sleep) were increased, compared to the placebo group. Further, the delta power in the first sleep cycle of the citrate group was increased to about 1.2 times the delta power in the first sleep cycle of the placebo group. It is shown that a combination drug of potassium citrate and sodium citrate hydrate can increase the percentage and duration of slow-wave sleep to overall sleep, reduce awakening after sleep onset (e.g., early-morning awakening), and improve the quality of sleep.

### Example 3

An open-label crossover test for aiming at confirming the effects of a combination formulation of potassium citrate, sodium citrate hydrate, and citric acid as well as a formulation of sodium bicarbonate (baking soda) on nocturnal urine output, frequency of nocturnal urination, and urine pH was performed on healthy males (aged 29 to 63 years) (the number of entries: 30). The subjects were given the following procedures 1) to 3) at intervals of 1 week or longer:
1) oral administration of two tablets each containing 231.5 mg of potassium citrate (C₆H₅K₃O₇·H₂O), 195.0 mg of sodium citrate hydrate (C₆H₅Na₃O₇·2H₂O), and 72.5 mg of anhydrous citric acid three times per day (morning, noon, evening) for 7 days (Group A: citric acid formulation administration group);
2) oral administration of four tablets each containing 500 mg of sodium bicarbonate three times per day (morning, noon, evening) for 7 days (Group B: baking soda administration group); and
3) no drug administration for 7 days (Group C: control).

On the last day of drug administration in each group, urine was collected for 24 hours using Urinemate (registered trademark) P (obtained from Sumitomo Bakelite Co., Ltd.), and the time, urine output, and urine pH were recorded for each urination. The "urine output between 22:00 (after) and early morning first urine" was defined as "nocturnal urine" and the "urine output between second urine and 22:00ʺ was defined as "diurnal urine".

Regarding the "nocturnal urine" and the "diurnal urine", the "urine output" and the "frequency of urination" were compared among the three groups. In addition, the relationship between the effects of "first urine" and "second urine" on pH and urine output was investigated.

On the urine collection day, the diet of the subjects was controlled, and the influence of diet (ingestion dose of salt, sugars, and protein, etc.) among the subjects was eliminated as much as possible.

The Wilcoxon signed rank test was used for statistical analysis.

The results are shown in Tables 1 to 3.

**[Table 1]**

| Influence of administration of citric acid formulation and baking soda formulation on nocturnal urine volume | | | | | | | |
|---|---|---|---|---|---|---|---|
| Group | No. | Diurnal urine (mL) | Nocturnal urine (mL) | 24-hour urine (mL) | Nocturnal urine/24-hour urine (%) | Diurnal urine-nocturnal urine (mL) | P value (diurnal vs nocturnal) |
| Control (C) | 27 | 991 ± 417 | 554 ± 370 | 1545 ± 571 | 36 ± 15 | 438 ± 544 | 0.0004 |
| Citric acid formulation (A) | 29 | 1024 ± 345 | 421 ± 184 | 1445 ± 384 | 30 ± 11 | 603 ± 397 | < 0.0001 |
| Baking soda formulation (B) | 26 | 1106 ± 392 | 499 ± 183 | 1605 ± 343 | 33 ± 13 | 607 ± 506 | < 0.0001 |
| P value (vs C) | | 0.3011 (A) | 0.0999 (A) | 0.6319 (A) | 0.0126 (A) | 0.0484 (A) | |
| | | 0.0814 (B) | 0.9944 (B) | 0.0814 (B) | 0.2382 (B) | 0.1283 (B) | |
| Mean ± SD | | | | | | | |

**[Table 2]**

| Influence of administration of citric acid formulation and baking soda formulation on frequency of urination | | | | | |
|---|---|---|---|---|---|
| Group | No. | Diurnal (frequency) | Nocturnal (frequency) | 24-hour urine (frequency) | P value (diurnal vs nocturnal) |
| Control (C) | 27 | 4.1 ± 1.2 | 2.2 ± 1.1 | 6.3 ± 1.7 | < 0.0001 |
| Citric acid formulation (A) | 29 | 4.1 ± 1.0 | 1.9 ± 0.7 | 6.0 ± 1.2 | < 0.0001 |
| Baking soda formulation (B) | 26 | 4.2 ± 1.4 | 1.8 ± 0.6 | 6.0 ± 1.5 | < 0.0001 |
| P value (vs C) | | 0.8160 (A) | 0.1309 (A) | 0.5024 (A) | |
| | | 0.8748 (B) | 0.0898 (B) | 0.4316 (B) | |
| Mean ± SD | | | | | |

**[Table 3]**

| Influence of administration of citric acid formulation and baking soda formulation on urine pH | | | | | | |
|---|---|---|---|---|---|---|
| Group | No. | Early morning first urine | | Early morning second urine | | P value (first urine vs second urine) |
| | | pH | ΔpH | pH | ΔpH | |
| Control (C) | 27 | 5.87 ± 0.54 | | 6.28 ± 0.61 | | 0.0055 |
| Citric acid formulation (A) | 29 | 6.31 ± 0.58 | 0.49 ± 0.69 | 6.91 ± 0.51 | 0.61 ± 0.64 | 0.0007 |
| Baking soda formulation (B) | 26 | 6.79 ± 0.61 | 0.94 ± 0.67 | 7.19 ± 0.42 | 0.95 ± 0.69 | 0.0055 |
| P value | | 0.0007 (A vs C) | 0.0012 | < 0.0001 (A vs C) | 0.0003 | |
| | | < 0.0001 (B vs C) | | < 0.0001 (B vs C) | | |
| | | 0.0013 (A vs B) | | 0.0006 (A vs B) | | |
| Mean ± SD | | | | | | |

The administration of the citric acid formulation (Group A) reduced the nocturnal urine output compared to a control group (Group C) (Table 1). Further, the administration of the citric acid formulation (Group A) reduced the percentage of nocturnal urine output in the daily urine output compared to the control group (Group C) (Table 1). The administration of the baking soda formulation (Group B) also reduced the nocturnal urine output compared to the control group (Group C) (Table 1). Furthermore, the administration of the baking soda formulation (Group B) reduced the percentage of nocturnal urine output in the daily urine output compared to the control group (Group C) (Table 1).

Regarding the frequency of urination, the administration of the citric acid formulation (Group A) did not change the frequency of diurnal urination, compared to the control group (Group C), but the frequency of nocturnal urination was decreased (Table 2). The administration of the baking soda formulation (Group B) did not change the frequency of diurnal urination, compared to the control group (Group C), but the frequency of nocturnal urination was decreased (Table 2).

The urine pH after administration of the citric acid formulation (Group A) and the urine pH after administration of the baking soda formulation (Group B) were significantly increased (alkalized), compared to the urine pH in the control group (Group C). As for the tested dose, the urine pH after administration of the baking soda formulation (Group B) was increased (alkalized), compared to the urine pH after administration of the citric acid formulation (Group A) (Table 3). However, the degree of urinary alkalinization effect of the citric acid formulation and the baking soda formulation was not reflected in the degree of the nocturnal urine output-reducing effect of both the formulations. Consequently, it was suggested that the effect of the citric acid formulation on reducing the nocturnal urine output was due not only to the urinary alkalinization but also to the effect of citric acid other than the urinary alkalinization effect of the citric acid formulation (Tables 1 and 3). Similarly, the degree of urinary alkalinization effect of the citric acid formulation and the baking soda formulation was not reflected in the degree of the nocturnal urination frequency-reducing effect of both the formulations. Therefore, it was suggested that the effect of the citric acid formulation on reducing the frequency of nocturnal urination was due not only to the urinary alkalinization but also to the effect of citric acid other than the urinary alkalinization effect of the citric acid formulation (Tables 2 and 3).

### Industrial Applicability

It is possible to provide a food composition or pharmaceutical composition useful in improving the quality of sleep in mammals, particularly humans.

## Claims

1. An agent for improving quality of sleep in mammals comprising, as an active ingredient, at least one substance selected from the group consisting of citric acid and a salt thereof.

2. The agent for improving quality of sleep according to claim 1 comprising citric acid as an active ingredient.

3. The agent for improving quality of sleep according to claim 1 or 2 comprising, as an active ingredient, an alkali metal salt of citric acid.

4. The agent for improving quality of sleep according to any one of claims 1 to 3 comprising, as an active ingredient, citric acid, sodium citrate or a hydrate thereof, and potassium citrate or a hydrate thereof.

5. The agent for improving quality of sleep according to any one of claims 1 to 4, wherein an improvement in quality of sleep is suppression in awakening after sleep onset.

6. The agent for improving quality of sleep according to any one of claims 1 to 5, wherein the improvement in quality of sleep is suppression in early-morning awakening.

7. The agent for improving quality of sleep according to any one of claims 1 to 6, wherein the improvement in quality of sleep is an improvement in sound sleep feeling.

8. The agent for improving quality of sleep according to any one of claims 1 to 7, wherein the improvement in quality of sleep is a promotion of slow-wave sleep.

9. The agent for improving quality of sleep according to any one of claims 1 to 8, wherein the improvement in quality of sleep is an increase in percentage of duration of NREM sleep stage 3 to duration of total sleep.

10. The agent for improving quality of sleep according to any one of claims 1 to 9, wherein the improvement in quality of sleep is a promotion of slow-wave sleep, which reduces a percentage of duration of NREM sleep stage 1 and increases a percentage of duration of NREM sleep stage 3.

11. The agent for improving quality of sleep according to any one of claims 1 to 10, wherein the improvement in quality of sleep is an improvement in quality of sleep in a first sleep cycle.

12. The agent for improving quality of sleep according to any one of claims 1 to 11, wherein the improvement in quality of sleep is an increase in delta power in the first sleep cycle.

13. The agent for improving quality of sleep according to any one of claims 1 to 12, wherein the improvement in quality of sleep is a promotion of deep sleep in the first sleep cycle.

14. The agent for improving quality of sleep according to any one of claims 1 to 13, wherein the improvement in quality of sleep is suppression in sleep disorders associated with frequent urination.

15. The agent for improving quality of sleep according to any one of claims 1 to 14, wherein the improvement in quality of sleep is suppression in awakening after sleep onset due to frequent urination.

16. The agent for improving quality of sleep according to any one of claims 1 to 15, wherein the agent is a food product.

17. The agent for improving quality of sleep according to any one of claims 1 to 16, wherein the agent is in a unit package form per serving, and is a food product containing 500 mg or more of at least one substance selected from the group consisting of citric acid and a salt thereof.

18. The agent for improving quality of sleep according to any one of claims 1 to 16, wherein the agent is in a unit package form per serving, and is a food product containing 500 mg or more and 2 g or less of at least one substance selected from the group consisting of citric acid and a salt thereof.

19. The agent for improving quality of sleep according to any one of claims 1 to 18, wherein the agent is a food product whose package, container, or instruction indicates an effect of improving the quality of sleep.

20. The agent for improving quality of sleep according to any one of claims 1 to 19, wherein the agent is a food product whose package, container, or instruction indicates an effect of increasing a percentage of duration of slow-wave sleep to duration of total sleep, reducing a frequency of awakening after sleep onset, or improving sound sleep feeling.
